(19) Europäisches Patentamt
European Patent Office
Office européen des brevets

(11) Veröffentlichungsnummer: **0 253 166 A2**

(12) **EUROPÄISCHE PATENTANMELDUNG**

(21) Anmeldenummer: **87109090.8**

(22) Anmeldetag: **24.06.87**

(51) Int. Cl.⁴: **C07D 405/06**, A61K 31/41, A61K 31/415

(30) Priorität: **07.07.86 DE 3622791**

(43) Veröffentlichungstag der Anmeldung: **20.01.88 Patentblatt 88/03**

(84) Benannte Vertragsstaaten: **AT BE CH DE ES FR GB GR IT LI LU NL SE**

(71) Anmelder: **HEUMANN PHARMA GMBH & CO**
**18-28 Heideloffstrasse**
**D-8500 Nürnberg(DE)**

(72) Erfinder: **Schickaneder, Helmut, Dr.Dipl.-Chem.**
**Moosaecker 25**
**D-8501 Eckental(DE)**
Erfinder: **Herter, Rolf, Dr.Dipl.-Chem.**
**Bayernstr. 42**
**D-8540 Schwabach(DE)**
Erfinder: **Vergin, Hartmut, Dr.Dipl.-Biochem.**
**Ebenreuther Str. 32**
**D-8500 Nuernberg 30(DE)**
Erfinder: **Engler, Heidrun, Dr.**
**Ringstr. 23**
**D-8501 Cadolzburg(DE)**
Erfinder: **Ahrens, Kurt Henning, Dr.**
**Praterstr. 9**
**D-8500 Nuernberg(DE)**

(74) Vertreter: **Kraus, Walter, Dr. et al**
**Patentanwälte Kraus, Weisert & Partner**
**Thomas-Wimmer-Ring 15**
**D-8000 München 22(DE)**

(54) **Neue 1,4-Piperazinderivate, Verfahren zu ihrer Herstellung, ihre Verwendung und diese enthaltendes Arzneimittel.**

(57) Es werden neue 1,4-Piperazinverbindungen der allgemeinen Formel

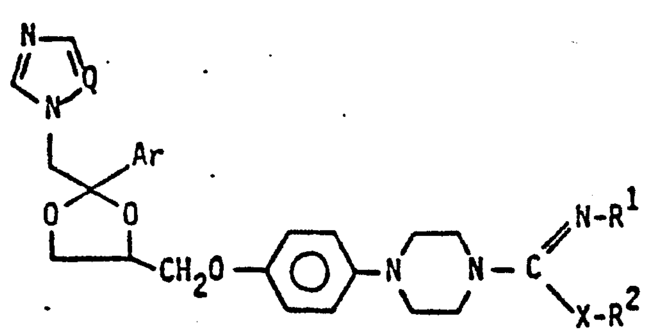

EP 0 253 166 A2

beschrieben, welche eine gegenüber den bekannten Verbindungen Miconazol und Ketoconazol verbesserte antimykotische Aktivität zeigen.

Die Erfindung betrifft neue Verbindungen mit antimykotischer Aktivität.

Es ist bereits eine Reihe antimykotisch aktiver Verbindungen bekannt, wie zum Beispiel Miconazol (DE-AS 19 40 388) oder Ketoconazol (DE-OS 28 04 096).

Der Erfindung liegt die Aufgabe zugrunde, neue Verbindungen mit verbesserter antimykotischer Aktivität zur Verfügung zu stellen. Diese Aufgabe wird durch die Erfindung gelöst.

Gegenstand der Erfindung sind neue 1,4-Piperazinverbindungen der allgemeinen Formel I

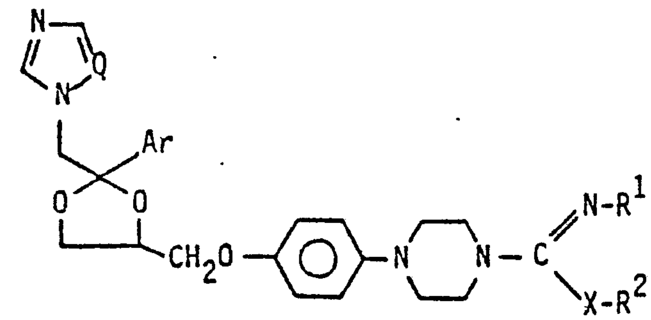

(I)

in der

Q für CH oder N steht,

Ar eine unsubstituierte oder mit bis zu 2 Halogenatomen substituierte Phenylgruppe bedeutet,

X für ein Sauerstoff-oder Schwefelatom steht,

$R^1$ ein Wasserstoffatom oder einen Niedrigalkylrest bedeutet und

$R^2$ einen $C_1$-$C_{10}$-Alkyl-, Cycloalkyl-, $C_1$-$C_{10}$-Alkenyl , Halogen-$C_1$-$C_{10}$ alkyl-, Hydroxy-$C_1$-$C_{10}$-alkyl-, $C_1$-$C_{10}$-Alkoxy-$C_1$-$C_{10}$-alkyl-, Di-$C_1$-$C_{10}$-alkylamino-$C_1$-$C_{10}$-alkyl-, Ar-Niedrigalkyl-oder Arylrest, der entweder unsubstituiert oder mit 1 bis 3 Substituenten aus der Gruppe Halogenatome, Niedrigalkyl-und Niedrigalkoxygruppen substituiert ist, bedeutet,

sowie die physiologisch annehmbaren Hydrate und Salze davon.

In der allgemeinen Formel I steht Q für CH oder N, wobei N bevorzugt wird.

Ar bedeutet eine unsubstituierte oder mit bis zu 2 Halogenatomen substituierte Phenylgruppe, wobei die Einfachsubstitution der Phenylgruppe bevorzugt wird. Als Halogensubstituenten kommen Fluor-, Chlor-und Bromatome in Frage, wobei Fluor-und Chloratome bevorzugt werden. Im Falle der Monosubstitution ist das Halogenatom vorzugsweise in 4-Stellung des Phenylrings angeordnet. Im Falle der Disubstitution sind die Substituenten vorzugsweise in 2-und 4-Stellung des Phenylrings angeordnet.

In der allgemeinen Formel I bedeutet X ein Sauerstoff-oder Schwefelatom, wobei ein Sauerstoffatom bevorzugt wird.

$R^1$ steht für ein Wasserstoffatom oder einen Niedrigalkylrest. Unter "Niedrigalkyl" wird hier ein Rest mit 1 bis 4 Kohlenstoffatomen verstanden. Beispiele für Niedrigalkylreste sind die Methyl-, Ethyl-, n-Propyl-, i-Propyl-, n-Butyl-, i-Butyl-, sec.-Butyl-und tert.-Butylgruppe. Gruppen mit 1 bis 3 Kohlenstoffatomen werden bevorzugt.

In der allgemeinen Formel I bedeutet der Rest $R^2$ eine $C_1$-$C_{10}$ Alkylgruppe, vorzugsweise eine $C_1$-$C_5$-Alkylgruppe und insbesondere eine Niedrigalkylgruppe, wie oben definiert. Bevorzugte Beispiele für solche Gruppen sind Methyl, Ethyl, Propyl, Butyl, Isopropyl und Isobutyl. Weiterhin bedeutet der Rest $R^2$ in der allgemeinen Formel I eine Cycloalkylgruppe mit 5 bis 12 Kohlenstoffatomen, wobei Ringe mit 5 bis 9 und mit 12 Kohlenstoffatomen bevorzugt werden. Bevorzugte Einzelbeispiele sind Cyclopentyl, Cyclohexyl, Cycloheptyl, Cyclooctyl und Cyclododecyl. Der Rest $R^2$ kann weiterhin eine $C_1$-$C_{10}$-Alkenylgruppe, vorzugsweise eine $C_1$-$C_5$-Alkenylgruppe und insbesondere eine Niedrigalkenylgruppe, wie oben definiert, sein. Bevorzugte Einzelbeispiele hierfür sind Allyl und Crotyl. Eine weitere Bedeutung für den Rest $R^2$ ist eine Halogen-$C_1$-$C_{10}$-alkylgruppe, vorzugsweise eine Halogen-$C_1$-$C_5$-alkylgruppe und insbesondere eine Halogenniedrigalkylgruppe, wie oben definiert. Als Halogenatome kommen Fluor-, Chlor-und Bromatome in Betracht. Die $C_1$-$C_{10}$-Alkylgruppe kann mit 1 bis 3 Halogenatomen substituiert sein, die am gleichen Kohlenstoffatom oder an unterschiedlichen Kohlenstoffatomen gebunden sein können. Eine Substitution der $C_1$-$C_{10}$-Alkylgruppe mit 1 bis 3 Chlor-oder Fluoratomen wird bevorzugt. Bevorzugte Einzelbeispiele hierfür sind 2-Fluorethyl, 2,2,2-Trifluorethyl, 2-Chlorpropyl und 4-Chlorbutyl. $R^2$ kann auch eine Hydroxy-$C_1$-$C_{10}$-alkylgruppe, vorzugsweise eine Hydroxy-$C_1$-$C_5$-alkylgruppe, insbesondere eine Hydroxyniedrigalkylgruppe, wie oben definiert, sein. Ein bevorzugtes Beispiel hierfür ist Hydroxyethyl. $R^2$ steht weiterhin für eine $C_1$-$C_{10}$-Alkoxy-$C_1$-$C_{10}$-alkylgruppe, vorzugsweise eine $C_1$-$C_5$ Alkoxy-$C_1$-$C_5$-alkylgruppe, insbesondere eine Niedrigal-

koxyniedrigalkylgruppe, wie oben definiert. Ein bevorzugtes Beispiel hierfür ist Methoxyethyl. Eine weitere Bedeutung für $R^2$ ist eine Di-$C_1$-$C_{10}$-alkylamino-$C_1$-$C_{10}$-alkylgruppe, vorzugsweise eine Di-$C_1$-$C_5$-alkylamino-$C_1$-$C_5$-alkylgruppe und insbesondere eine Diniedrigalkylaminoniedrigalkylgruppe. Das Stickstoffatom der Di-C1-$C_{10}$-alkylaminogruppe kann mit gleichen oder verschiedenen $C_1$-$C_{10}$-Alkylgruppen substituiert sein. Ein bevorzugtes Beispiel für eine solche Gruppe ist Dimethylaminoethyl. Schließlich steht $R^2$ noch für eine Aralkylgruppe, vorzugsweise die Benzylgruppe. Die Aralkylgruppe kann entweder unsubstituiert sein oder mit 1 bis 3 Substituenten aus der Gruppe Halogenatome, wie oben angegeben, Niedrigalkylgruppen, wie oben definiert, und Niedrigalkoxygruppen, wie oben definiert, substituiert sein. Als Substituenten der Aralkylgruppe werden Niedrigalkylgruppen, wie insbesondere Methyl und Ethyl, und Halogenatome, wie Fluor und Chlor, sowie Alkoxygruppen, wie Methoxy, bevorzugt.

Eine bevorzugte Gruppe der erfindungsgemäßen Verbindungen ist dadurch gekennzeichnet, daß X für ein Sauerstoffatom und $R^1$ für ein Wasserstoffatom steht, wobei die Substituenten Q, Ar und $R^2$ die oben angegebenen Bedeutungen haben.

Die erfindungsgemäßen Verbindungen der allgemeinen Formel I können wie folgt hergestellt werden:

a) Verbindungen der allgemeinen Formel I, bei denen $R^1$ für Wasserstoff steht und $R^2$, Q, Ar und X die oben angegebenen Bedeutungen haben, können hergestellt werden

$a_1$) durch Umsetzung einer Verbindung der allgemeinen Formel II

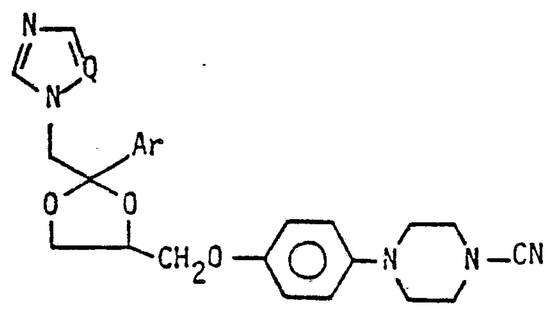

(II)

in der Q und Ar die oben angegebenen Bedeutungen haben, mit einer Verbindung der allgemeinen Formel III

$R^2XH$ (III)

worin $R^2$ und X die oben angegebenen Bedeutungen haben.

Die Umsetzung erfolgt in üblicher Weise in Gegenwart von Mineralsäuren, wie zum Beispiel Salzsäure, und unter Zusatz eines inerten Lösungsmittels, wie zum Beispiel eines chlorierten Kohlenwasserstoffs, beispielsweise Chloroform.

Die Umsetzung im basischen Milieu wird jedoch bevorzugt. Als Lösungsmittel dient eine Verbindung der allgemeinen Formel

$R^2XH$

wobei $R^2$ die oben angegebene Bedeutung hat, oder ein Gemisch aus der Verbindung $R^2XH$ und einem inerten Lösungsmittel, beispielsweise einem Ether, wie THF, einem Kohlenwasserstoff, wie Toluol, oder einem chlorierten Kohlenwasserstoff, wie Dichlorethan. Die Verbindung $R^2XH$ wird vorzugsweise in mindestens stöchiometrischer Menge eingesetzt. Als Base können das Natriumsalz der Verbindung $R^2XH$ oder ein Alkali-oder Erdalkalihydroxid oder -cyanid verwendet werden. Die Base wird in einem Mengenverhältnis von 0.01 bis 3 Mol-Äquivalenten, bevorzugt 0.1 Mol-Äquivalenten, bezogen auf die Verbindung II, eingesetzt. Die Reaktionstemperatur kann zwischen 20°C und Rückflußtemperatur des jeweils verwendeten Mediums variiert werden.

Die bei der obigen Reaktion als Ausgangsverbindung eingesetzte Verbindung der allgemeinen Formel II kann beispielsweise dadurch erhalten werden, daß man eine Verbindung der allgemeinen Formel IX

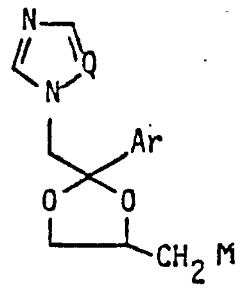

(IX)

worin Q und Ar die oben angegebenen Bedeutungen haben und M den Methansulfonsäure-oder Toluolsulfonsäureester bedeutet,

mit einer Verbindung der allgemeinen Formel XII

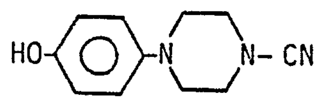

(XII)

umsetzt. Die Umsetzung erfolgt auf übliche Weise mit Hilfe einer Base, wie Natriumhydrid oder -carbonat, in einem inerten Lösungsmittel, wie DMF, und bei erhöhter Temperatur, vorzugsweise bei 60 bis 120°C.

Die Ausgangsverbindung II kann alternativ auch dadurch erhalten werden, daß man eine Verbindung der allgemeinen Formel IV

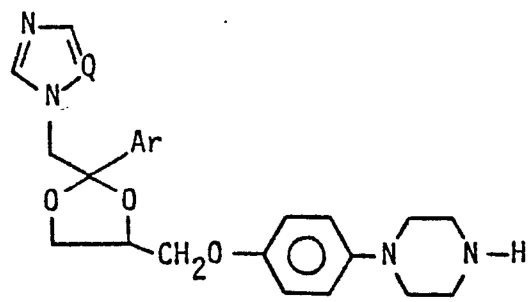

(IV)

in der Q und Ar die oben angegebenen Bedeutungen haben, mit Chlorcyan oder Bromcyan umsetzt. Die Reaktion wird vorzugsweise in einem inerten Lösungsmittel, wie Dichlormethan, Tetrahydrofuran etc., durchgeführt. Als Base dient hierbei ein tertiäres Amin, vorzugsweise Triethylamin, oder eine anorganische Base, wie Calciumhydroxid oder Kaliumcarbonat.

Eine weitere Synthesemöglichkeit für die Verbindung der allgemeinen Formel II besteht in der Umsetzung einer Verbindung der allgemeinen Formel XIII

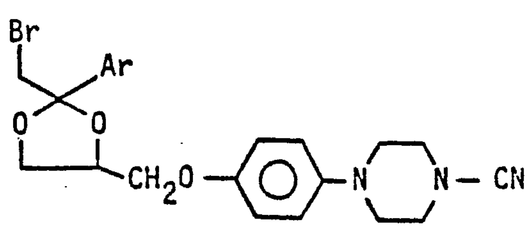

(XIII)

worin Ar die oben angegebene Bedeutung hat, mit einer Verbindung der a119emeinen Formel XIV

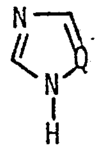

(XIV)

worin Q die oben angegebene Bedeutung besitzt.

Die Reaktion kann beispielsweise in Dimethylformamid oder Dimethylsulfoxid bei einer Temperatur von 60 bis 180°C, vorzugsweise 130 bis 150°C, durchgeführt werden. Als Base kommen insbesondere Natriumhydrid oder eine anorganische Base, wie Kaliumcarbonat, in Betracht.

Die bei diesem Syntheseweg verwendete Verbindung der allgemeinen Formel XIII kann beispielsweise durch Umsetzung einer Verbindung der allgemeinen Formel XV

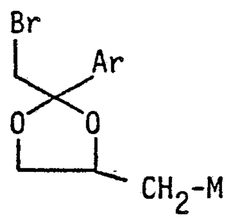

(XV)

worin Ar die oben angegebene Bedeutung hat und M den Methansulfonsäure-oder Toluolsulfonsäureester bedeutet,

mit einer Verbindung der allgemeinen Formel XII

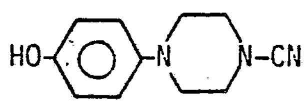

(XII)

hergestellt werden. Die Umsetzung kann bei den gleichen Bedingungen durchgeführt werden, wie sie oben im Zusammenhang mit der Umsetzung der Verbindung der allgemeinen Formel IX mit der Verbindung der Formel XII beschrieben wurden.

oder

$a_2$) durch Umsetzung einer Verbindung der allgemeinen Formel IV

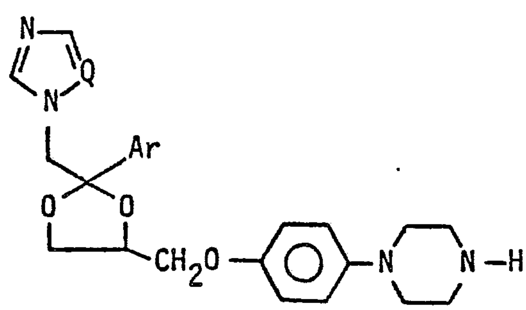

(IV)

worin Q und Ar die oben angegebenen Bedeutungen haben, mit einer Verbindung der allgemeinen Formel V

$R^2XCN$ (V)

worin $R^2$ und X die oben angegebenen Bedeutungen haben.

Die Ausgangsverbindung der allgemeinen Formel VI, die nach literaturbekannten Methoden in einfacher Weise zugänglich ist, wird hierbei in äquimolarer Menge mit der Verbindung der allgemeinen Formel IV in einem inerten Lösungsmittel umgesetzt. Als Lösungsmittel kommen Ether, wie Tetrahydrofuran, Kohlenwasserstoffe, wie Toluol, und chlorierte Kohlenwasserstoffe, wie Dichlorethan, in Betracht. Die Reaktion kann bei Temperaturen von -20°C bis +40°C, vorzugsweise 0 bis 10°C, durchgeführt werden. Die Aufarbeitung erfolgt durch Einengen des Reaktionsgemisches und Kristallisation des Rückstands.

b) Verbindungen der allgemeinen Formel I, bei denen $R^1$ für Wasserstoff steht, $R^2$ wie oben definiert ist, jedoch mit der Maßgabe, daß $R^2$ nicht für einen Arylrest steht, X für ein Schwefelatom steht und Q und Ar die oben angegebenen Bedeutungen haben, können hergestellt werden durch Umsetzung einer Verbindung der allgemeinen Formel IV

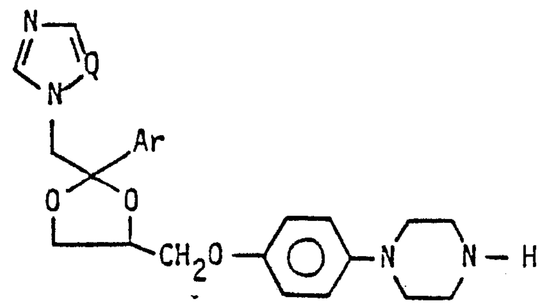

(IV)

worin Q und Ar die oben angegebenen Bedeutungen haben, mit einer Verbindung der allgemeinen Formel VI

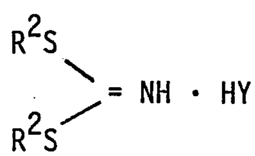

(VI)

worin $R^2$ die oben angegebene Bedeutung hat und HY für eine anorganische Säure steht. Die anorganische Säure HY kann zum Beispiel HCl, HBr, HJ oder $(H_2SO_4)_{1/2}$ sein.

Die Umsetzung kann in einem inerten Lösungsmittel, beispielsweise einem Ether, wie Tetrahydrofuran, einem chlorierten Kohlenwasserstoff, wie Dichlorethan, oder vorzugsweise einem Nitril, insbesondere Acetonitril, durchgeführt werden. Eine Reaktionstemperatur von 60 bis 100°C wird bevorzugt. Die Aufarbeitung erfolgt entweder durch Einengen des Reaktionsgemisches, wobei das Produkt als Salz der Säure HY anfällt, oder durch vorheriges Entfernen der Säure HY mittels einer wäßrig-alkalischen Lösung und Einengen der organischen Phase, wobei das Produkt als freie Base erhalten wird.

c) Verbindungen der allgemeinen Formel I, bei denen $R^1$, $R^2$, Q und Ar die oben angegebenen Bedeutungen haben und X für ein Schwefelatom steht, können durch Umsetzung einer Verbindung der allgemeinen Formel VII

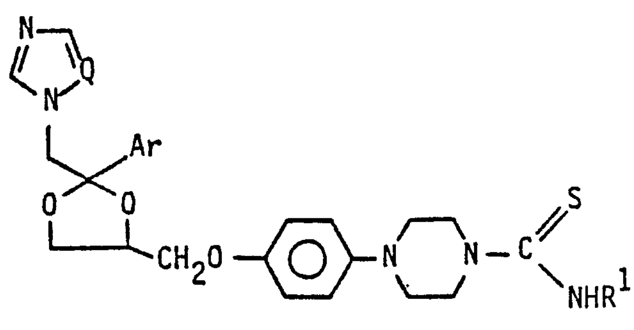

(VII)

worin Q, Ar und $R^1$ die oben angegebenen Bedeutungen haben, mit einer Verbindung der allgemeinen Formel VIII

$R^2Z$ (VIII)

worin $R^2$ die oben angegebene Bedeutung hat und Z für eine Austrittsgruppe steht, hergestellt werden. Wenn in der Verbindung der Formel VIII der Rest $R^2$ eine aliphatische Gruppe ist, kann Z zum Beispiel die Bedeutung Halogen, wie Chlor, Brom und Jod, Methansulfonat, Benzolsulfonat oder Toluolsulfonat haben.

Die Umsetzung erfolgt bei 0 bis 150°C in einem inerten Lösungsmittel, das beispielsweise ein Ether, wie Tetrahydrofuran, ein Kohlenwasserstoff, wie Toluol, ein chlorierter Kohlenwasserstoff, wie Chloroform oder 1,2-Dichlorethan, oder ein Nitril, wie Acetonitril, sein kann. Als Base kann ein tertiäres Amin oder ein anorganisches basisches Salz, wie Calciumhydroxid oder Calciumcarbonat, zugesetzt werden. Wird während der Reaktion auf den Zusatz einer Base verzichtet, dann erfolgt die Aufarbeitung in Gegenwart einer wäßrig-alkalischen Lösung.

Ist in der Verbindung der allgemeinen Formel VIII der Rest $R^2$ jedoch eine Arylgruppe, muß Z eine Diazoniumgruppe bedeuten. In diesem Falle erfolgt dann die Umsetzung vorzugsweise in einem Zweiphasensystem Wasser/Chlorkohlenwasserstoff, wobei als Chlorkohlenwasserstoff 1,2-Dichlorethan bevorzugt wird. In diesem Fall wird dem Reaktionsgemisch noch 0.01 bis 0.1 Mol-Äquivalent eines Phasentransferkatalysators, wie $(n\text{-}Bu)_4NHSO_4$, zugesetzt. Die Reaktionstemperatur beträgt 10°C bis Rückflußtemperatur des verwendeten organischen Lösungsmittels. Die Aufarbeitung und Isolierung erfolgt in üblicher Weise.

Die bei dieser Variante als Ausgangsverbindung verwendete Verbindung der allgemeinen Formel VII kann in an sich bekannter Weise aus einer Verbindung der allgemeinen Formel IV

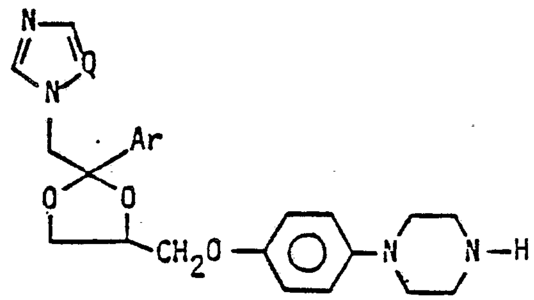

(IV)

worin Q und Ar die oben angegebenen Bedeutungen haben, und einem Isothiocyanat der allgemeinen Formel XVI

$R^1NCS$ (XVI)

worin $R^1$ die oben angegebene Bedeutung hat, hergestellt werden.

d) Verbindungen der allgemeinen Formel I, bei denen $R^1$ für ein Wasserstoffatom steht, X für ein Sauerstoffatom steht und $R^2$, Q und Ar die oben angegebenen Bedeutungen haben, können durch Umsetzung einer Verbindung der allgemeinen Formel IX

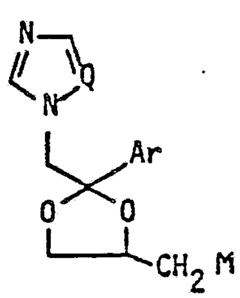

(IX)

worin Q und Ar die oben angegebenen Bedeutungen haben und M den Methansulfonsäure-oder Toluolsulfonsäureester bedeutet,

mit einer Verbindung der allgemeinen Formel X

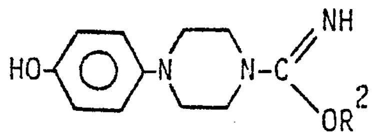

(X)

worin $R^2$ die oben angegebene Bedeutung hat, hergestellt werden. Als reaktiver Esterrest wird zum Beispiel ein Methansulfonat-, Benzolsulfonat-oder 4-Toluolsulfonatrest verwendet. Die Umsetzung kann bei den gleichen Bedingungen vorgenommen werden, wie sie oben im Zusammenhang mit der Umsetzung der Verbindung der allgemeinen Formel IX mit der Verbindung der allgemeinen Formel XII beschrieben wurden.

Die bei dieser Variante als Ausgangsverbindung eingesetzte Verbindung der allgemeinen Formel X ist ihrerseits leicht aus der Verbindung XII

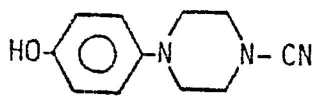

(XII)

und der Verbindung der allgemeinen Formel III

$R^2XH$ (III)

zugänglich.

Die Umsetzung kann bei den gleichen Bedingungen erfolgen, wie sie oben im Zusammenhang mit der Verfahrensvariante $a_1$) beschrieben wurde.

e) Verbindungen der allgemeinen Formel I, bei denen $R^1$ für einen Niedrigalkylrest steht, X für ein Sauerstoffatom steht und $R^2$, Q und Ar die oben angegebenen Bedeutungen haben, können durch Umsetzung einer Verbindung der allgemeinen Formel Ia

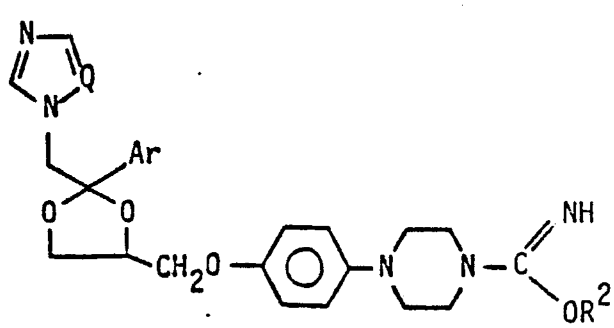

(Ia)

worin Q, Ar und $R^2$ die oben angegebenen Bedeutungen haben, mit einer Verbindung der allgemeinen Formel XI

$R^1Z$ (XI)

worin $R^1$ für einen Niedrigalkylrest, wie oben definiert, steht und Z eine Austrittsgruppe bedeutet, hergestellt werden. Die Austrittsgruppe Z kann beispielsweise ein Halogenatom, wie Chlor, Brom und Jod, oder eine Methansulfonat-, Benzolsulfonat-oder Toluolsulfonatgruppe sein. Die Umsetzung erfolgt in einem inerten Lösungsmittel, beispielsweise einem Ether, wie Tetrahydrofuran, einem Kohlenwasserstoff, wie Toluol,

einem Halogenkohlenwasserstoff, wie Dichlorethan, einem Nitril, wie Acetonitril, oder einem Amid, wie Dimethylformamid.

Die Reaktionstemperatur kann 20°C bis Rückflußtemperatur des Lösungsmittels betragen. Dem Reaktionsgemisch wird vorzugsweise noch mindestens eine stöchiometrische Menge einer Base zugesetzt, wobei die Base entweder ein tertiäres Amin oder eine anorganische Base, wie Natriumhydrid oder Kaliumcarbonat, sein kann.

Die nach den Verfahrensvarianten a) bis e) erhaltenen Verbindungen werden in üblicher Weise isoliert und gereinigt, beispielsweise durch chromatographische Methoden, Umkristallisation, Extraktion etc.

Wie oben bereits ausgeführt, können die in den Stufen a) bis e) erhaltenen Verbindungen mit geeigneten Säuren, wie oben beschrieben, in ihre physiologisch annehmbaren Salze umgewandelt werden.

Die erfindungsgemäßen Verbindungen zeigen sowohl eine wesentlich bessere antimykotische In-vitro-Wirksamkeit als auch eine bessere therapeutisch nutzbare In-Vivo-Wirksamkeit als zum Beispiel Miconazol oder Ketoconazol.

Die neuen Verbindungen sind zur äußerlichen, aber auch zur oralen Behandlung von Pilzinfektionen an Mensch und Tier geeignet.

Als Darreichungsformen kommen beispielsweise Tabletten, Kapseln, Suspensionen, Lösungen, Gelees, Cremes, Salben etc. in Betracht.

Die therapeutisch wirksame Verbindung ist in pharmazeutischen Zubereitungen vorzugsweise in einer Konzentration von 0.01 bis 90 Gew.-% der Gesamtmischung vorhanden.

Die Anwendungskonzentration für Lösungen, Gelees, Cremes oder Salben beträgt im allgemeinen zwischen 0.1 und 4 Gew.-%.

Bei oraler Verabreichung in Form von Tabletten oder Kapseln beträgt die Tagesdosis des Wirkstoffs etwa 1.0 bis 50.0 mg/kg Körpergewicht in Mischung mit einem gebräuchlichen Trägerstoff.

Die folgenden Beispiele erläutern die Herstellung der erfindungsgemäßen Verbindungen und ihrer Vorprodukte.

Beispiel I

(±)-cis-4[4-[[2-(2-,4-Dichlorphenyl)-2-(IH-I,2.4-triazol-I-ylmethyl)-1,3-dioxolan-4-yl]-methyoxy]-phenyl]-piperazin-I-carbimidsäure-[2-(N,N-dimethylamino)ethyl]-ester

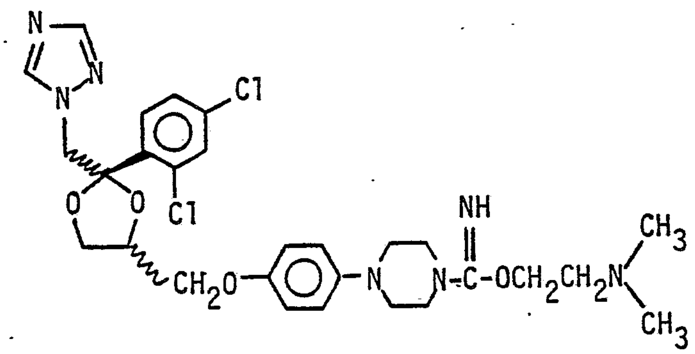

I.80 g (3.5 mmol) (±)-cis-1-Cyano-4-[4-[[2-(2,4-dichlorphenyl) -2-(IH-I-,2,4-triazol-I-ylmethyl)-I,3-dioxolan-4-yl]-methoxy]-phenyl] -piperazin werden in einer Lösung aus 8 mg (0.35 mmol) Natrium in I0 ml N,N-Dimethylaminoethanol und 20 ml Toluol unter Rückfluß erhitzt. Nach 2 h engt man im Vakuum ein, versetzt mit 20 ml $H_2O$ und extrahiert dreimal mit je 25 ml Ethylacetat.

Man zieht das Lösungsmittel im Vakuum ab und erhält 2.5 g eines Öls. Dieses wird in wenig Ethylacetat gelöst. Nach Abkühlen im Eisbad wird von der ausgefallenen Titelverbindung abgesaugt.

Ausbeute: 1.45 g (69 % d.Th.)

Cremefarbene Kristalle vom Schmp. I02 - I04° C.

Rf: 0.26 (Kieselgel; Dichlormethan/Methanol/$NH_3$(25 %) 90/9/1)

$C_{28}H_{35}Cl_2N_7O_4$ (M: 604.5)

Ber.: C 55.63 H 5.84 N 16.22
Gef.: C 55.96 H 5.80 N 16.09

| | |
|---|---|
| $^1$H-NMR-Daten: ($CDCl_3$, TMS als interner Standard) | $\delta$ = 2.32 (s) 6 H, |
| | 2.67 (t) 2 H, |
| | 3.08 (m) 4 H, |
| | 3.51 (m) 5 H, |
| | 3.87 (m) 3 H, |
| | 4.19 (t) 2 H, |
| | 4.38 (m) 1 H, |
| | 4.4 - 5.1 (breit) 1 H, austauschbar mit $D_2O$, |
| | 4.82 (s) 2 H, |
| | 6.88 (m) 4 H, |
| | 7.2 - 7.8 (m) 3 H, |
| | 7.93 1 H, |
| | 8.26 1 H ppm. |

Herstellung von (±)-cis-1-Cyano-4-[4-[[2-(2,4-dichlorphenyl) -2-(1H-1-,2,4-triazol-1-ylmethyl)-1.3-dioxolan-4-yl]-methoxy]-phenyl] -piperazin

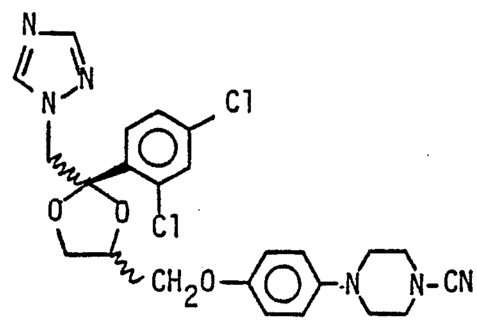

Weg A

Eine Lösung von 19.62 g (40 mmol) (±)-cis-1-[4-[[2-(2,4-Dichlorphenyl)-2-(1H-1-,2,4-triazol-1-ylmethyl)-1,3-dioxolan-4-yl]-methoxy] -phenyl]-piperazin und 4.25 g (42 mmol) Triethylamin in 70 ml Dichlormethan werden unter Eiskühlung tropfenweise mit 4.45 g (42 mmol) Bromcyan in 70 ml Dichlormethan versetzt. Man rührt 12 h bei RT, gießt auf 16 g $K_2CO_3$ in 150 ml $H_2O$ und extrahiert mit Dichlormethan.

Nach Abziehen des Lösungsmittels im Vakuum verbleibt ein Öl, das durch Behandeln mit Ethylacetat kristallisiert. Das primäre Kristallisat wird aus wenig Methanol umkristallisiert.

Ausbeute: I5.4 g (= 74 % d.Th.)
Farbloses Pulver vom Schmelzpunkt I35° C.
Rf = 0.4I (Ethylacetat/Methanol 95/5)

$C_{24}H_{24}Cl_2N_6O_3$ (M: 5I4.4)

Ber.: C 55.93 H 4.69 N I6.3I
Gef.: C 56.04 H 4.7I N I6.29

| | |
|---|---|
| $^1$H-NMR-Daten: (CDCl$_3$, TMS als interner Standard) | δ = 3.13 (m) 4 H, 3.37 (m) 4 H, 3.45 - 4.1 (m) 4 H, 4.38 (m) 1 H, 4.83 (s) 2 H, 6.92 (m) 4 H, 7.2 - 7.75 (m) 3 H, 7.93 (s) 1 H, 8.28 (s) 1 H ppm. |

Weg B

Zu I77 mg (5.9 mmol) Natriumhydrid (80 %-ige Dispersion in Mineralöl) in I0 ml DMF tropft man eine Lösung von 996 mg (4.9 mmol) I-Cyano-4-(4-hydroxyphenyl)-piperazin in ebenfalls I0 ml DMF. Dann gibt man 2.30 g (4.9 mmol) (±)-cis-[2-(2,4-Dichlorphenyl)-2-(IH-I-,2,4-triazol-I-ylmethyl)-I,3-dioxolan-4-yl]-methy-4-toluolsulfonat in I2 ml DMF hinzu und erhitzt 45 Min. auf 90° C. Das Reaktionsgemisch wird im Vakuum eingeent, die org. Phase mit $H_2O$ gewaschen und über $MgSO_4$ getrocknet. Nach Abkühlen des Lösungsmittels verbleibt ein rötliches Öl, das aus Ethylacetat kristallisiert.

Umkristallisation aus Methanol ergibt die Titelverbindung als weißes Pulver.
Ausbeute: I.2 g (= 48 % d.Th.)

Weg C

(±)-cis-[2-(Brommethyl)-2-(2,4-dichlorphenyl)-l,3-dioxolan-4-ylmethyl] -4-toluolsufonat

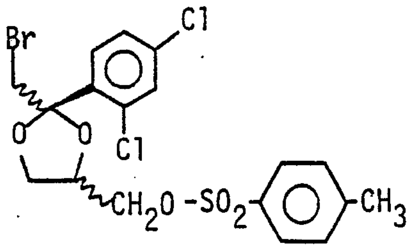

Zu einer Lösung von l02.4 g (299 mmol) (±)-cis-2-(Brommethyl)-2-(2,4-dichlorphenyl)-l,3-dioxolan-4-methanol in 4OO ml Dichlormethan und 73 ml Pyridin tropft man eine Lösung von 6O.3 g (3lO mmol) 4-Toluolsulfonsäurechlorid in 3OO ml Dichlormethan.
Man rührt l2 h bei RT, wäscht das Reaktionsgemisch dann nacheinander mit Wasser, verd. wäßriger HCl und wäßriger $NaHCO_3$-Lösung und trocknet die organische Phase über Magnesiumsulfat. Nach Abziehen des Lösungsmittels im Vakuum verbleibt ein hellgelbes Öl, das rein genug ist für weitere Umsetzungen.

Ausbeute: l32.O g (89 % d.Th.)

Rf: O.7O (Kieselgel; Ethylacetat/Petrolether 6O/4O)

(±)-cis-l-[4-[[2-(Brommethyl)-2-(2,4-dichlorphenyl)-l,3-dioxolan-4-yl] -methoxy]-phenyl]-4-cyano-piperazin

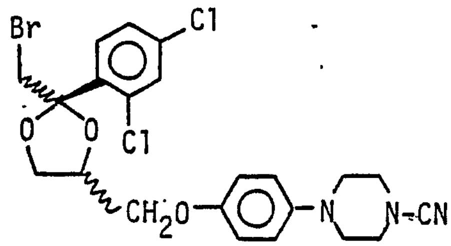

Zu 675 mg (22.5 mmol) NaH (8O%-ige Dispersion in Mineralöl) in 2O ml DMF tropft man nacheinander eine Lösung von 4.O7 g (2O mmol) l-Cyano-4-(4-hydroxyphenyl)-piperazin und eine Lösung von 9.92 g (2O mmol) der oben erhaltenen Verbindung. Das Reaktionsgemisch wird 2O h auf 8O - 9O° C erhitzt.
Man gießt auf Wasser und extrahiert mit Dichlormethan. Die organische phase wird im Vakuum eingeengt und der Rückstand an Kieselgel chromatographiert. (Ethylacetat/Petrolether 5O-7O° C, 6O/4O).

Ausbeute: 3.6 g (34 % d.Th.)

Farbloser Feststoff vom Schmelzpunkt l38.5 - l39.5° C.

Rf: O.59 (Kieselgel; Ethylacetat/Petrolether 6O/4O)

$C_{22}H_{22}BrCl_2N_3O_3$ (M: 527.3)

| | |
|---|---|
| $^1H$-NMR-Daten: | δ = 3.12 (m) 4 H, |
| ($CDCl_3$, TMS als | 3.37 (m) 4 H, |
| interner Standard) | 3.65 - 4.6 (m) 7 H, |
| | 6.92 (s) 4 H, |
| | 7.15 - 7.8 (m) 3 H ppm. |

(±)-cis-l-Cyano-[4-[[2-(2,4-dichlorphenyl)-2-(lH-l-,2,4-triazol-l-ylmethyl)-l,3-dioxolan-4-yl]-methoxy]-phenyl]-piperazin

Zu 340 mg (ll.4 mmol) Natriumhydrid (80 %-ige Dispersion in Mineralöl) in lO ml DMF tropft man eine Lösung von 0.8 g (ll.4 mmol) l,2,4-Triazol bei Raumtemperatur. Nach beendeter $H_2$-Entwicklung gibt man l,5 g (2.9 mmol) der oben erhaltenen Verbindung zu und erhitzt l2 h auf l40° C. Man gießt auf Wasser und extrahiert mit Dichlorethan. Nach Abziehen des Lösungsmittels im Vakuum verbleibt ein dunkelbraunes, zähes Öl. Durch Zusatz von wenig Methanol kristallisiert die Titelverbindung als schwach beige gefärbter Feststoff.
Die spektroskopischen und chromatographischen Daten stimmen mit den weiter oben berichteten überein.

Beispiel 2

(±)-cis-4-[4-[[2-(2,4-Dichlorphenyl)-2-(lH-l-,2,4-triazol-l-ylmethyl)-l,3-dioxolan-4-yl]-methoxy]-phenyl]-piperazin-l-carbimidsäure-(2-propenyl)-ester

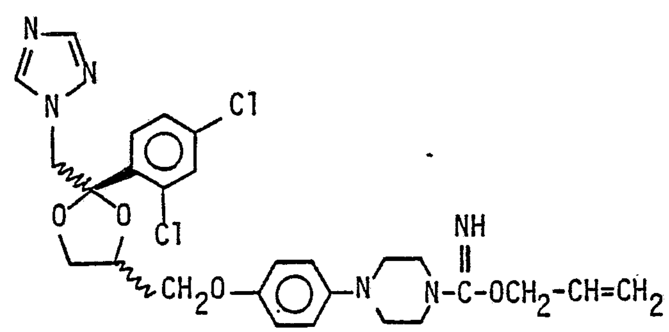

Die Herstellung erfolgt analog Beispiel l, jedoch mit 2 Propenol.

Farblose Kristalle vom Schmelzpunkt l05 - l07° C
Rf: 0.52 (Kieselgel: Ethylacetat/Methanol/Triethylamin 85/l0/5)

$C_{27}H_{30}Cl_2N_6O_4$ (M: 573.5)

| | |
|---|---|
| $^1$H-NMR-Daten: | $\delta$ = 3.10 (m) 4 H, |
| ($CDCl_3$, TMS als | 3.53 (m) 5 H, |
| interner Standard) | 3.89 (m) 3 H, |
| | 4.38 (m) 1 H, |
| | 4.63 (d) 2 H, |
| | 4.83 (s) 2 H, |
| | 4.2 - 5.1 (breit), 1 H, austauschbar mit $D_2O$, |
| | 5.1 - 5.6 (m) 2 H, |
| | 5.7 - 6.4 (m) 1 H, |
| | 6.93 (m) 4 H, |
| | 7.2 - 7.75 (m) 3 H, |
| | 7.94 (s) 1 H, |
| | 8.27 (s) 1 H ppm. |

Beispiel 3

(±)-cis-4-[4-[[2-(2,4-Dichlorphenyl)-2-(lH-l,2,4-triazol-l-ylmethyl) -l,3-dioxolan-4-yl]-methoxy]-phenyl]-piperazin-l-carbimidsäureethylester

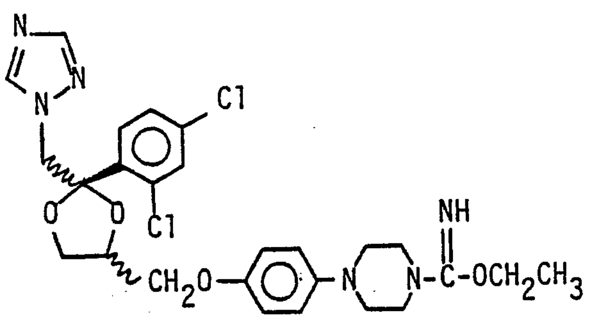

Die Herstellung erfolgt analog Beispiel l. jedoch mit Ethanol.
Cremefarbenes Pulver vom Schmelzpunkt l02 - l03° C.

Rf: O.33 (Kieselgel; Ethylacetat/Methanol/Triethylamin 85/lO/5)

$C_{26}H_{30}Cl_2N_6O_4$(M: 56l.5)

$^1$H-NMR-Daten: (CDCl$_3$, TMS als interner Standard)

δ = 1.32 (t) 3 H,
3.07 (m) 4 H,
3.5 (m) 5 H,
3.88 (m) 3 H,
4.11 (q) 2 H,
4.33 (m) 1 H,
4.24 (verbr. s) 1 H, austauschbar mit $D_2O$,
4.83 (s) 2 H,
6.88 (m) 4 H,
7.2 - 7.75 (m) 3 H,
7.94 (s) 1 H,
8.26 (s) 1 H ppm.

Beispiel 4

(±)-cis-4-[4-[[2-(2,4-Dichlorphenyl)-2-(IH-I-,2,4-triazol-I-ylmethyl) -I,3-dioxolan-4-yl]-methoxy]-phenyl]-piperazin-I-carbimidsäurebenzylester

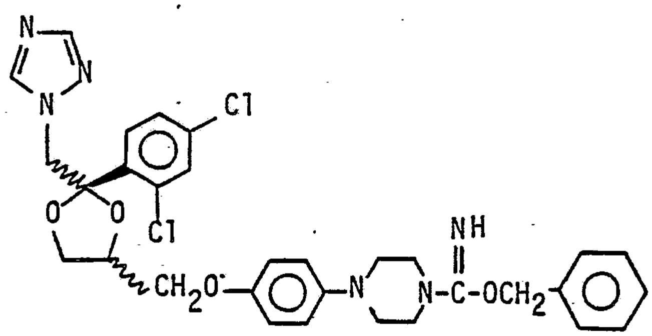

Die Herstellung erfolgt analog Beispiel I, jedoch mit Benzylalkohol.
Farblose Kristalle vom Schmelzpunkt IOI - IO3° C

Rf: 0.37 (Kieselgel; Ethylacetat/Methanol/Triethylamin 9O/5/5)

$C_{31}H_{32}Cl_2N_6O_4$ (M: 623.5)

$^1$H-NMR-Daten: (CDCl$_3$, TMS als interner Standard)

$\delta$ = 3.1 (m) 4 H,
3.55 (m) 5 H,
3.9 (m) 2 H,
4.21 (verbr. s) 1 H, austauschbar mit $D_2O$,
4.4 (m) 1 H,
4.83 (s) 2 H,
5.19 (s) 2 H,
6.90 (m) 4 H,
7.25 - 7.75 (m) 3 H,
7.44 (s) 5 H,
7.95 (s) 1 H,
8.27 (s) 1 H ppm.

Beispiel 5

(±)-cis-4-[4-[[2-(2,4-Dichlorphenyl)-2-(IH-I-,2,4-triazol-I-ylmethyl) -I,3-dioxolan-4-yl]-methoxy]-phenyl]-piperazin-I-carbimidsäurecyclohexylester

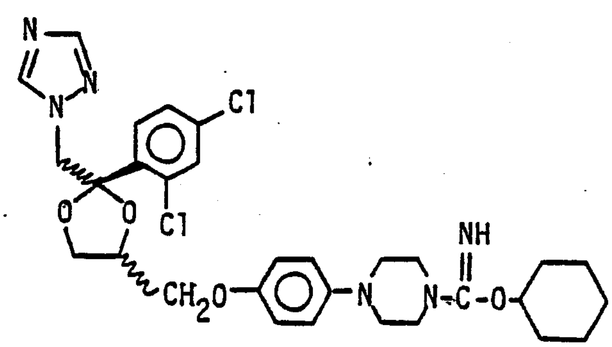

Die Herstellung erfolgt analog Beispiel I, jedoch mit Cyclohexanol.

Farblose Kristalle vom Schmelzpunkt 99 - IOI° C

Rf: O.4O (Kieselgel, Ethylacetat/Methanol/Triethylamin 9O/5/5)

$C_{30}H_{35}Cl_2N_6O_4$ (M: 6I4.6)

| | |
|---|---|
| $^{1}$H-NMR-Daten: | δ = 1.15 - 2.25 (m) 10 H, |
| ($CDCl_3$, TMS als | 3.08 (m) 4 H, |
| interner Standard) | 3.5 (m) 5 H, |
| | 3.88 (m) 4 H, 1 H austauschbar mit $D_2O$, |
| | 4.38 (m) 1 H, |
| | 4.63 (m) 1 H, |
| | 4.82 (s) 2 H, |
| | 6.88 (m) 4 H, |
| | 7.2 - 7.8 (m) 3 H, |
| | 7.94 (s) 1 H, |
| | 8.29 (s) 1 H ppm. |

Beispiel 6

(±)-cis-4-[4-[[2-(2,4-Dichlorphenyl)-2-(lH-l-,2,4-triazol-l-ylmethyl)-l,3-dioxolan-4-yl]-methoxy]-phenyl]-piperazin-l-carbimidsäurebutylester

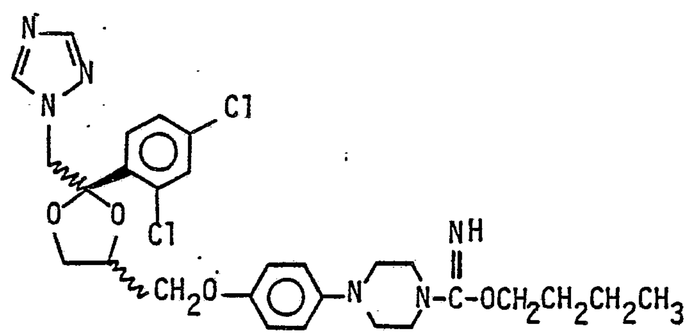

Die Herstellung erfolgt analog Beispiel l, jedoch mit l-Butanol.

Schwach gelb gefärbte Kristalle vom Schmelzpunkt 89 - 92° C.

Rf: 0.38 (Kieselgel: Ethylacetat/Methanol/Triethylamin 85/l0/5)

$C_{28}H_{34}Cl_2N_6O_4$ (M: 589,5)

$^1$H-NMR-Daten: (CDCl$_3$, TMS als interner Standard)

δ = 0.93 (m) 3 H,
1.1 - 1.9 (m) 4 H,
3.07 (m) 4 H,
3.50 (m) 5 H,
3.85 (m) 3 H,
4.03 (t) 2 H,
4.33 (m) 2 H, 1 H austauschbar mit $D_2O$,
4.80 (s) 2 H,
6.85 (m) 4 H,
7.2 - 7.75 (m) 3 H,
7.92 (s) 1 H,
8.24 (s) 1 H ppm.

Beispiel 7

(±)-cis-4-[4-[[2-(2,4-Dichlorphenyl)-2-(lH-l-,2,4-triazol-l-ylmethyl) -l,3-dioxolan-4-yl]-methoxy]-phenyl]-piperazin-l-thiocarbimidsäurebutylester

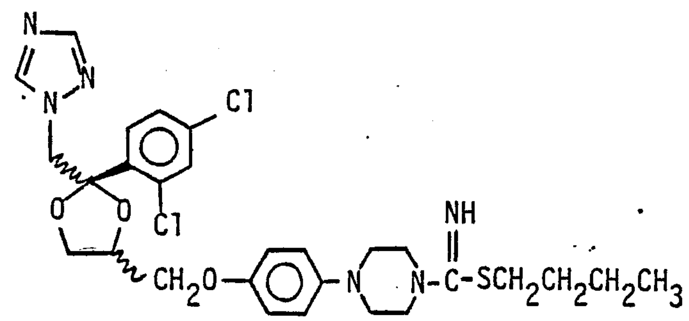

Die Herstellung erfolgt analog Beispiel l. jedoch mit 1-Butanthiol.

Beigefarbener, kristalliner Feststoff vom Schmelzpunkt l22 - l24° C.

Rf: O.47 (Kieselgel; Ethylacetat/Methanol/Triethylamin 9O/5/5)

$C_{28}H_{34}Cl_2N_6O_3S$ (M: 6O5,6)

| | |
|---|---|
| $^1$H-NMR-Daten: | $\delta$ = 0.94 (m) 3 H, |
| (TMS als | 1.1 - 1.9 (m) 4 H, |
| interner Standard) | 2.81 (t) 2 H, |
| | 3.12 (m) 4 H, |
| | 3.4 - 4.2 (m) 8 H, |
| | 4.38 (m) 1 H, |
| | 4.5 - 5.2 (breit) 1 H, austauschbar mit $D_2O$, |
| | 4.82 (s) 2 H, |
| | 6.87 (m) 4 H, |
| | 7.2 - 7.8 (m) 3 H, |
| | 7.93 (s) 1 H, |
| | 8.26 (s) 1 H ppm. |

Beispiel 8

(±)-cis-4-[4-[[2-(2,4-Dichlorphenyl)-2-(lH-l-,2,4-triazol-l-ylmethyl) -l,3-dioxolan-4-yl]-methoxy]-phenyl]-piperazin-l-carbimidsäuremethylester-fumarat

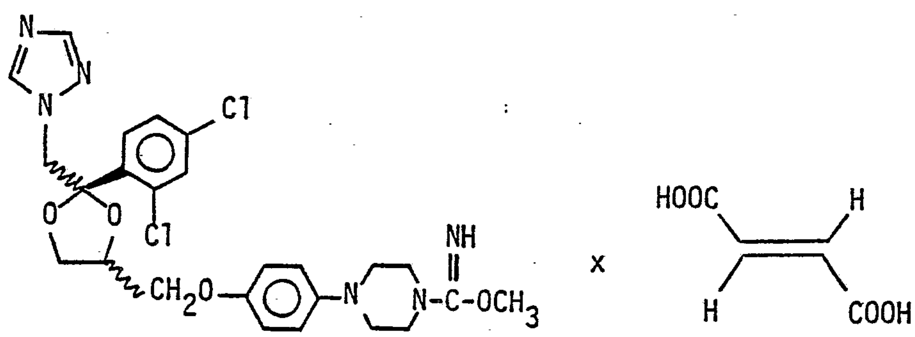

25.0 g (48.5 mmol) (±)-cis-l-Cyano-4-[4-[[2-(2,4-dichlorphenyl)-2-(lH-l-,2,4-triazol-l-ylmethyl)-l,3-dioxolan-4-yl]-methoxy]-phenyl]-piperazin werden in einer Lösung aus ll2 mg (4.9 mmol) Natrium in 65 ml Methanol und l25 ml Toluol unter Rückfluß erhitzt.

Nach 4 h engt man im Vakuum ein, nimmt in 200 ml $CH_3OH/CH_2Cl_2$ (20/80 V/V) auf und wäscht mit wenig Wasser. Man zieht das Lösungsmittel im Vakuum ab und erhält l6.05 g eines Öls.

Dieses wird in 200 ml Aceton in der Wärme gelöst und mit einer Lösung von 3.57 g (30.8 mmol) Fumarsäure in 50 ml EtOH versetzt.

Nach Abkühlen auf RT saugt man vom ausgefallenen Fumarat ab und wäscht mit wenig Aceton.

Ausbeute: l5.l g (= 47 % d.Th.)

Farbloser Feststoff vom Schmelzpunkt l60 - l63° C (Differentialthermoanalyse).

Rf = 0.32 (Ethylacetat/Methanol/Triethylamin 85/l0/5)

$C_{29}H_{32}Cl_2N_6O_8$ (M: 663,5)

Ber.: C 52.49 H 4.82 N 12.67
Gef.: C 52.19 H 4.81 N 12.39

| | |
|---|---|
| $^1$H-NMR-Daten: ($d_6$-DMSO, TMS als interner Standard) | $\delta$ = 3.12 (m) 4 H, |
| | 3.3 - 4.15 (m) 8 H, |
| | 3.99 (s) 3 H, |
| | 4.39 (t) 1 H, |
| | 4.86 (s) 2 H, |
| | 6.63 (s) 2 H, |
| | 6.96 (s) 4 H, |
| | 7.35 - 7.80 (m) 3 H, |
| | 7.93 (s) 1 H, |
| | 8.48 (s) 1 H, |
| | 10.33 (s) 3 H (austauschbar mit $D_2O$) ppm. |

Beispiel 9

(±)-cis-4-[4-[[2-(2,4-Dichlorphenyl)-2-(lH-l-,2,4-triazol-l-ylmethyl) -l,3-dioxolan-4-yl]-methoxy]-phenyl]-piperazin-l-carbimidsäureisopropylester-fumarat

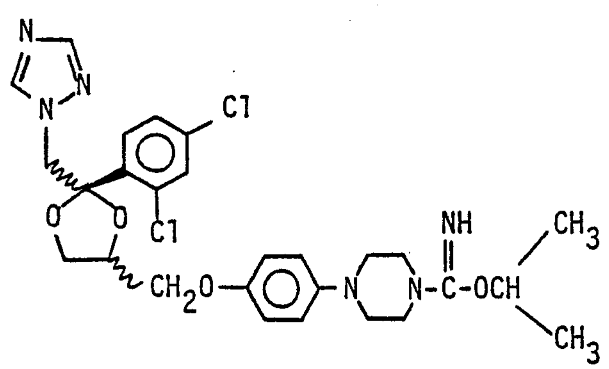

x

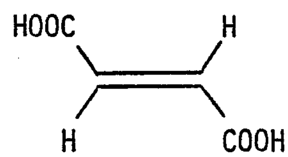

Die Herstellung erfolgt analog Beispiel 8, jedoch mit Isopropanol.

Weißes. amorphes Pulver

Rf: 0.35 (Kieselgel; Ethylacetat/Methanol/Triethylamin 85/10/5)

$C_{31}H_{36}Cl_2N_6O_8$ (M: 691.6)

$^1$H-NMR-Daten: (d6-DMSO, TMS als interner Standard)

δ = 1.43 (d) 6 H,
3.2 (m) 4 H,
3.8 (m) 8 H,
4.4 (m) 1 H,
4.9 (m) 2 H,
5.1 (m) 1 H,
6.70 (s) 2 H,
7.03 (m) 4 H,
7.3 - 8.3 (m) 6 H, davon 3 H austauschbar mit $D_2O$
7.90 (s) 1 H,
8.48 (s) 1 H ppm.

Beispiel IO

(±)-cis-4-[4-[[2-(2,4-Dichlorphenyl)-2-(IH-I-,2,4-triazol-I-ylmethyl)-I,3-dioxolan-4-yl]-methoxy]-phenyl]-piperazin-I-carbimidsäure-(2-methoxyethyl)-ester-fumarat

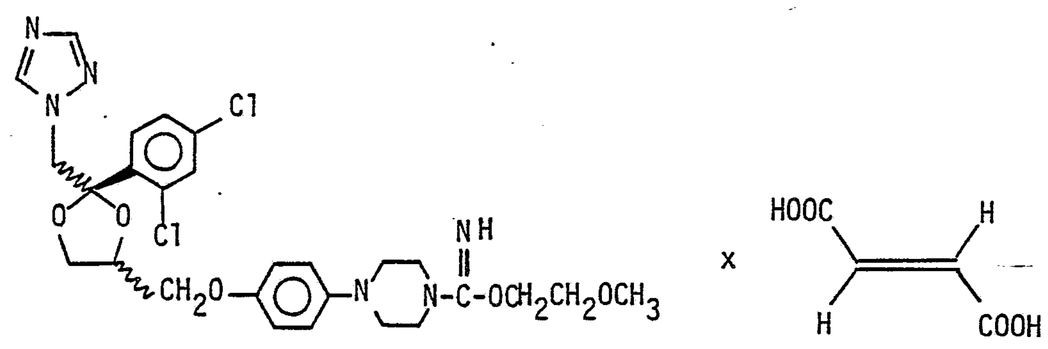

Die Herstellung erfolgt analog Beispiel 8, jedoch mit 2-Methoxyethanol.

Weißes, amorphes Pulver.

Rf: O.27 (Kieselgel; Ethylacetat/Methanol/Triethylamin 85/IO/5)

$C_{31}H_{36}Cl_2N_6O_9$ (M: 7O7.6)

| | |
|---|---|
| $^1$H-NMR-Daten: (d$_6$-DMSO, TMS als interner Standard) | δ = 3.12 (m) 4 H, |
| | 3.33 (s) 3 H, |
| | 3.4 - 4.15 (m) 10 H, |
| | 4.42 (m) 3 H, |
| | 4.85 (verbr. s) 2 H, |
| | 6.60 (s) 2 H, |
| | 6.7 - 7.35 (m) 7 H, 3 H austauschbar mit $D_2O$, |
| | 7.4 - 7.7 (m), |
| | 7.92 (s) 1 H, |
| | 8.48 (s) 1 H ppm. |

Beispiel II

(±)-cis-4-[4-[[2-(2,4-Dichlorphenyl)-2-(imidazol-l-ylmethyl)-l,3-dioxolan-4-yl]-methoxy]-phenyl]-piperazin-l-l-carbimidsäuremethylester

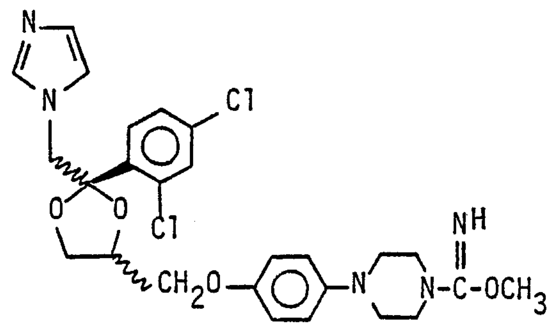

Die Herstellung erfolgt analog Beispiel I, jedoch mit (±)-cis-l-Cyano-4-[4-[[2-(2,4-dichlorphenyl)-2-imidazol-l-ylmethyl)-l,3-dioxolan-4-yl]-methoxy]-phenyl]-piperazin und Methanol.

Farbloses Pulver vom Schmelzpunkt l32.5 - l34.5° C

Rf: O.22 (Kieselgel: Ethylacetat/Methanol/Triethylamin 85/lO/5)

$C_{26}H_{29}Cl_2N_5O_4$ (M: 546.5)

| | |
|---|---|
| $^1$H-NMR-Daten: | δ = 3.1 (m) 4 H, |
| ($CDCl_3$, TMS als interner Standard) | 3.2 - 4.0 (m)3 H, 1 H austauschbar mit $D_2O$, |
| | 3.77 (s) 3 H, |
| | 4.37 (m) 1 H, |
| | 4.47 (m) 2 H, |
| | 6.87 (m) 4 H, |
| | 7.01 (m) 2 H, |
| | 7.2 - 7.7 (m) 4 H ppm. |

Herstellung von (±)-cis-l-Cyano-4-[4-[[2-(2,4-dichlorphenyl)-2-(imidazol-l-ylmethyl)-l,3-dioxolan-4-yl]-methoxy]-phenyl]-piperazin

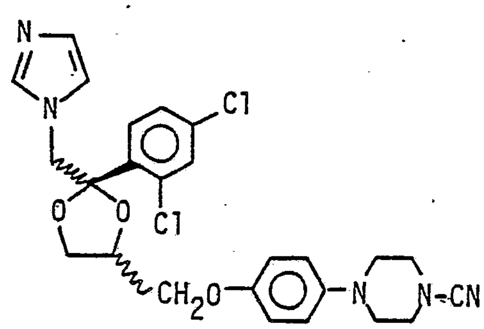

Eine Lösung von 4.07 g (20 mmol) l-Cyano4-(4-hydroxyphenyl)-piperazin in 20 ml DMF tropft man zu einer Suspension von 675 mg (22.5 mmol) Natriumhydrid (80%-ige Suspension in Mineralöl) in 25 ml DMF. Nach beendeter Gasentwicklung gibt man 8.l5 g (20 mmol) (±)-cis-[2-(2,4-dichlorphenyl)-2-(imidazol-l-ylmethyl)-l,3-dioxolan-4-yl]-methy-4-toluolsulfonat zu und erhitzt l0 h auf 90° C. Man gießt auf Wasser, extrahiert mit l,2-Dichlorethan und wäscht die organische Phase mit Wasser. Nach Abziehen des Lösungsmittels im Vakuum verbleibt ein Öl, das durch Filtration über Kieselgel (Eluent: Ethylacetat/Ethanol 90:10) gereinigt wird. Die Titelverbindung kann in der Kälte aus wenig Methanol kristallisiert werden.

Ausbeute: 5.3 g (52 % d.Th.)
Farbloses Pulver vom Schmelzpunkt ll8 - l20° C.

Rf: 0.60 (Kieselgel: Dichlormethan/Methanol 90/10)

$C_{25}H_{25}Cl_2N_5O_3$ (M: 5l4.4)

| | |
|---|---|
| $^1$H-NMR-Daten: | $\delta$ = 3.13 (m) 4 H, |
| ($CDCl_3$, TMS als | 3.39 (m) 4 H, |
| interner Standard) | 3.55 - 4.1 (m) 4 H, |
| | 4.36 (m) 1 H, |
| | 4.46 (m) 2 H, |
| | 6.86 (m) 4 H, |
| | 7.02 (m) 2 H, |
| | 7.2 - 7.8 (m) 4 H ppm. |

Beispiel I2

(±)-cis-4-[4-[[2-(2,4-Fluorphenyl)-2-(IH-I-,2,4-triazol-I-ylmethyl)-I,3-dioxolan-4-yl]-methoxy]-phenyl]-piperazin-I-carbimidsäuremethylester

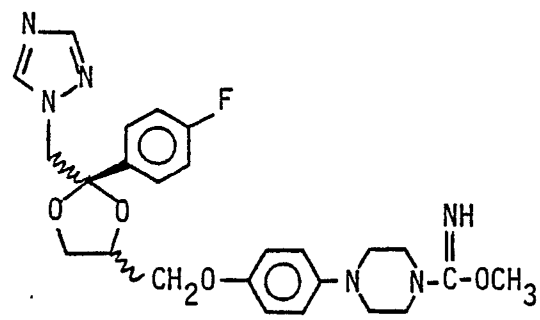

Die Herstellung erfolgt analog Beispiel I jedoch mit (±)-cis-I-Cyano-4-[4-[[2-(2,4-fluorphenyl)-2-(IH-I-,2,4-triazol-I-ylmethyl)-I,3-dioxolan-4-yl] -methoxy]-phenyl]-piperazin und Methanol. Farbloser Feststoff vom Schmelzpunkt I64 - I66° C

Rf: O.4O (Kieselgel; Dichlormethanol/Methanol/$NH_3$ (25 %) 9O/9/I

$C_{25}H_{29}FN_4O_4$ (M: 468,5)

| | |
|---|---|
| $^{1}$H-NMR-Daten: | $\delta$ = 3.07 (m) 4 H, |
| ($CDCl_3$, TMS als | 3.52 (m) 5 H, |
| interner Standard) | 3.77 (s) 3 H, |
| | 3.83 (m) 3 H, |
| | 4.2 - 4.9 (breit) 1 H, austauschbar mit $D_2O$, |
| | 4.36 (m) 1 H, |
| | 4.53 (s) 2 H, |
| | 6.84 (m) 4 H, |
| | 7.10 (m) 2 H, |
| | 7.54 (m) 2 H, |
| | 7.94 (s) 1 H, |
| | 8.24 (s) 1 H ppm. |

Herstellung von (±)-cis-l-Cyano-4-[4-[[2-(4-fluorphenyl)-2-(lH-l-,2,4-triazol-l-ylmethyl)-1,3-dioxolan-4-yl]-methoxy]-phenyl]-piperazin

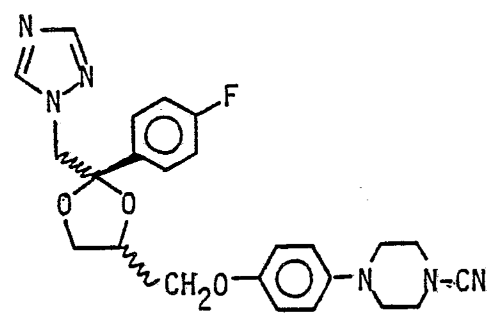

Zu einer Aufschlämmung von 0.66 g (22 mmol) Natriumhydrid (80%-ige Dispersion in Mineralöl) in 60 ml DMF tropft man eine Lösung von 3.66 g (l8 mmol) l-Cyano-4-(4-hydroxyphenyl)-piperazin in 40 ml DMF.

Nach beendeter Gasentwicklung gibt man 6.0 g (l5 mmol) (±)-cis-[2-(4-Fluorphenyl)-2-(lH-l,2,4-triazol-l-ylmethyl)-l,3-dioxolan-4-yl]-methyl-methansulfonat zu und erhitzt 5 h auf 90° C.
Nach dem Abkühlen gießt man auf 300 ml Eiswasser. Die Titelverbindung kristallisiert als beigefarbener Feststof, der aus wenig Ethylacetat umkristallisiert wird.
Ausbeute: 4.9 g (70 % d.Th.)

Farbloses Pulver vom Schmelzpunkt l52 - l53° C

Rf: 0.50 (Kieselgel: Ethylacetat/Methanol 90/10)

$C_{24}H_{25}F_4N_6O_3$ (M: 464.5)

| | |
|---|---|
| $^1$H-NMR-Daten: | $\delta$ = 3.12 (m) 4 H, |
| ($CDCl_3$, TMS als | 3.39 (m) 5 H, |
| interner Standard) | 3.83 (m) 3 H, |
| | 4.35 (m) 1 H, |
| | 4.53 (s) 2 H, |
| | 6.86 (m) 4 H, |
| | 7.09 (m) 2 H, |
| | 7.53 (s) 2 H, |
| | 7.93 (s) 1 H, |
| | 8.25 (s) 1 H ppm. |

Beispiel I3

(±)-cis-4-[4-[[2-(2,4-Dichlorphenyl)-2-(IH-I,2,4-triazol-I-ylmethyl)-I,3-dioxolan-4-yl]-methoxy]-phenyl]-piperazin-I-carbimidsäurephenylester

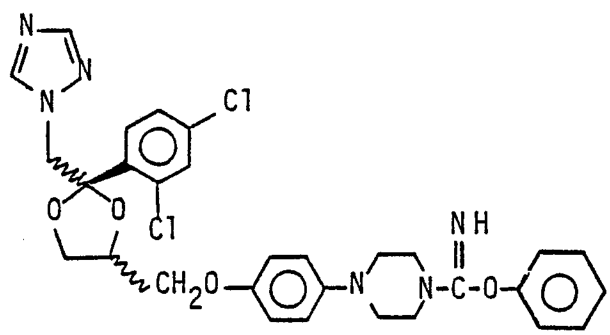

I.47 g (3 mmol) (±)-cis-1-[4-[[2-(2,4-Dichlorphenyl)-2-(IH-1,2,4-triazol-I-ylmethyl)-I,3-dioxolan-4-yl]-methoxy]-phenyl]-piperazin in IO ml THF werden bei RT unter Rühren zu einer Lösung von O.39 g (3.3 mmol) Phenylcyanat getropft. Das Reaktionsgemisch erwärmt sich auf 3O° C. Man rührt noch I h nach, engt im Vakuum ein und nimmt den öligen Rückstand in 3O ml Ethylacetat auf. Durch Rühren der Lösung im Eisbad kristallisiert die Titelverbindung als farbloser Feststoff.

Ausbeute: I.5O g (82 % d.Th.)

Farblose Kristalle vom Schmelzpunkt I52.5 - I53° C.

Rf: O.5O (Kieselgel; Ethylacetat/Methanol/Triethylamin 9O/5/5)

$C_{30}H_{30}Cl_2N_6O_4$ (M: 6O9,5)

| | |
|---|---|
| $^1$H-NMR-Daten: | δ = 3.17 (m) 4 H, |
| ($CDCl_3$, TMS als | 3.4 - 4.15 (m) 8 h, |
| interner Standard) | 4.38 (m) 2 H, 1 H austauschbar mit $D_2O$, |
| | 4.83 (s) 2 H, |
| | 6.7 - 7.8 (m) 12 H, |
| | 7.95 (s) 1 H, |
| | 8.27 (s) 1 H ppm. |

Beispiel I4

(±)-cis-4-[4-[[2-(2,4-Dichlorphenyl)-2-(IH-I,2,4-triazol-I-ylmethyl)-I,3-dioxolan-4-yl]-methoxy]-phenyl]-piperazin-I-carbimidsäure-(3-methylphenyl)-ester

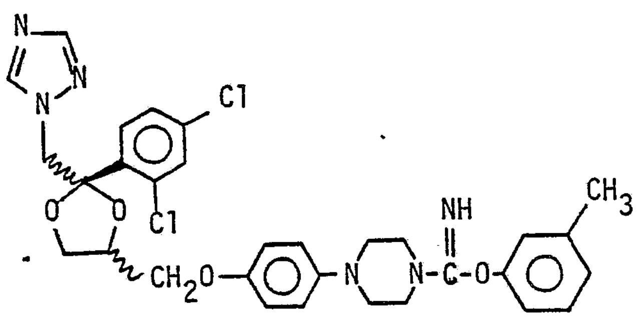

Die Herstellung erfolgt analog Beispiel I3, jedoch mit 3-Methylphenylcyanat.

Ausbeute: I.28 9 (68 % d.Th.)

Leicht gelbliche Kristalle vom Schmelzpunkt I63 - I65° C.

Rf: 0.46 (Kieselgel: Ethylacetat/Methanol/Triethylamin 90/5/5)

$C_{31}H_{32}Cl_2N_6O_4$ (M: 623.5)

| | |
|---|---|
| $^1$H-NMR-Daten: | δ = 2.37 (s) 3 H, |
| ($CDCl_3$, TMS als | 3.13 (m) 4 H, |
| interner Standard) | 3.4 - 4.1 (m) 8 H, |
| | 4.33 (m) 2 H, 1 H austauschbar mit $D_2O$, |
| | 4.81 (s) 2 H, |
| | 6.7 - 7.8 11 H, |
| | 7.93 (s) 1 H, |
| | 8.25 (s) 1 H ppm. |

Beispiel 15

(±)-cis-4-[4-[[2-(2,4-Dichlorphenyl)-2-(1H-1,2,4-triazol-1-ylmethyl)-1,3-dioxolan-4-yl]-methoxy]-phenyl]-piperazin-1-carbimidsäure-(4-methoxyphenyl)-ester

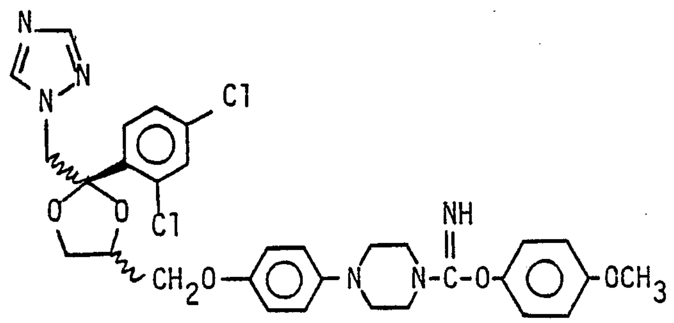

Die Herstellung erfolgt analog Beispiel 13, jedoch mit 4-Methoxyphenylcyanat.

Farblose Kristalle vom Schmelzpunkt 176.5 - 177.5° C

Rf: 0.47 (Kieselgel; Ethylacetat/Methanol/Triethylamin 85/10/5)

$C_{31}H_{32}Cl_2N_6O_5$ (M: 639,5)

| | |
|---|---|
| $^1$H-NMR-Daten: (CDCl$_3$, TMS als interner Standard) | δ = 2.37 (s) 3 H,<br>3.4 - 4.1 (m) 9 H, austauschbar mit $D_2O$,<br>3.83 (s) 3 H,<br>4.38 (m) 1 H,<br>4.82 (s) 2 H,<br>6.90 (m) 4 H,<br>7.01 (m) 4 H,<br>7.2 - 7.7 (m) 3 H,<br>7.93 (s) 1 H,<br>8.25 (s) 1 H ppm. |

Beispiel 16

(±)-cis-4-[4-[[2-(2,4-Dichlorphenyl)-2-(lH-l,2,4-triazol-l-ylmethyl)-l,3-dioxolan-4-yl]-methoxy]-phenyl]-piperazin-l-carbimidsäure-(2,2,2-trifluorethyl)-ester

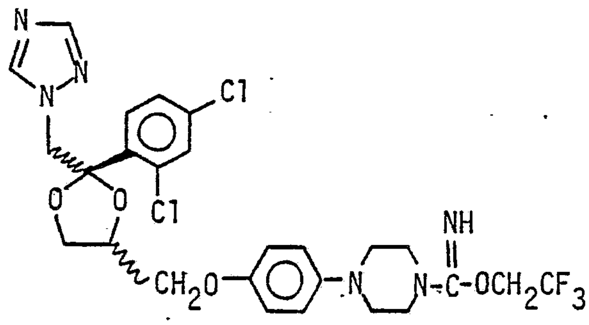

Die Herstellung erfolgt analog Beispiel l3, jedoch mit 2,2,2-Trifluorethylcyanat.

Farblose Kristalle vom Schmelzpunkt l34.5 - l35° C (Ethylacetat/Diethylether).

Rf: 0.52 (Kieselgel: Ethylacetat/Methanol/Triethylamin 90/5/5)

$C_{26}H_{27}F_3Cl_2N_6O_4$ (M: 6l5,4)
Ber.: C 50.74 H 4.42 N 13.66
Gef.: C 50.82 H 4.52 N 13.90

| | |
|---|---|
| $^1$H-NMR-Daten: | $\delta$ = 3.1 (m) 4 H, |
| ($CDCl_3$, TMS als | 3.5 (m) 5 H, |
| interner Standard) | 3.85 (m) 3 H, |
| | 4.4 (m) 1 H, |
| | 4.60 (q) 2 H, |
| | 4.83 (s) 2 H, |
| | 4.2 - 5,5 (breit) 1 H, austauschbar mit $D_2O$, |
| | 6.90 (m) 4 H, |
| | 7.2 - 7.8 (m) 3 H, |
| | 7.95 (s) 1 H, |
| | 8.27 (s) 1 H ppm. |

Beispiel l7

(±)-cis-4-[4-[[2-(2,4-Dichlorphenyl)-2-(lH-l,2,4-triazol-l-ylmethyl)-l,3-dioxolan-4-yl]-methoxy]-phenyl]-piperazin-l-carbimidsäure-(4-chlorphenyl)-ester

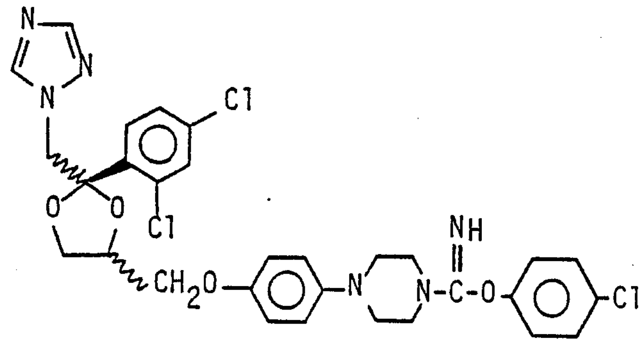

Die Herstellung erfolgt analog Beispiel l3, jedoch mit 4-Chlorphenylcyanat.

Farblose Kristalle vom Schmelzpunkt l6l - l63° C.

Rf: O.54 (Kieselgel: Ethylacetat/Methanol/Triethylamin 9O/5/5)

$C_{30}H_{29}Cl_3N_6O_4$ (M: 644.O)

| | |
|---|---|
| $^1$H-NMR-Daten: (CDCl$_3$, TMS als interner Standard) | δ = 3.14 (m) 4 H,<br>3.4 - 4.15 (m) 8 H,<br>4.40 (m) 1 H,<br>4.7 - 5.3 (breit) 1 H, austauschbar mit $D_2O$,<br>4.84 (s) 2 H,<br>6.7 - 7.8 (m) 11 H,<br>7.95 (s) 1 H,<br>8.27 (s) 1 H ppm. |

Beispiel l8

(±)-cis-4-[4-[[2-(2,4-Dichlorphenyl)-2-(lH-l,2,4-triazol-l-ylmethyl)-l,3-dioxolan-4-yl]-methoxy]-phenyl]-piperazin-l-thiocarbimidsäuremethylester

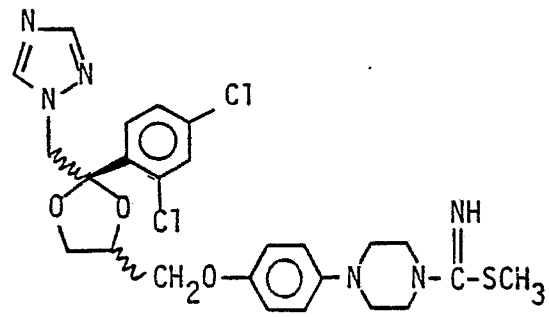

10.0 g (20.4 mmol) (±)-cis--[4-[[2-(2,4-Dichlorphenyl)-2-(lH-l-,2,4-triazol-l-ylmethyl)-l,3-dioxolan-4-yl]-methoxy]-phenyl]-piperazin werden in 100 ml Acetonitril aufgeschlämmt und mit 5.08 g (20.4 mmol) Iminodithiokohlensäuredimethylester-Hydrojodid versetzt. Das Reaktionsgemisch wird 2 h zum Rückfluß erhitzt, abgekühlt und auf 200 ml einer 10%-igen wäßrigen Kaliumcarbonatlösung gegossen.
Die Extraktion mit Ethylacetat ergibt 15.0 g einer halbkristallinen Masse, die an Kieselgel chromatographiert wird (Laufmittel: Ethylacetat/Methanol/Triethylamin 90/5/5). Anschließend wird die Titelverbindung aus tert.-Butylmethylether umkristallisiert.

Ausbeute: 4.95 g (43 % d.Th.)

Farblose Kristalle vom Schmelzpunkt 160 - 161° C. -

Rf: 0.43 (Kieselgel Ethylacetat/Methanol/Triethylamin 90/5/5)

$C_{25}H_{28}Cl_2Ni_6O_3S$ (M: 563,5)

| | |
|---|---|
| $^1$H-NMR-Daten: | $\delta$ = 2.32 (s) 3 H, |
| ($CDCl_3$, TMS als | 3.08 (m) 4 H, |
| interner Standard) | 3.4 - 4.15 (m) 8 H, |
| | 4.37 (m) 1 H, |
| | 4.81 (m) 2 H, |
| | 5.8 - 6.3 (breit), 1 H, austauschbar mit $D_2O$, |
| | 6.88 (m) 4 H, |
| | 7.15 - 7.8 (m) 3 H, |
| | 7.83 (s) 1 H, |
| | 8.28 (s) 1 H ppm. |

Beispiel 19

(±)-cis-4-[4-[[2-(2,4-Dichlorphenyl)-2-(IH-I,2,4-triazol-I-ylmethyl)-I,3-dioxolan-4-yl]-methoxy]-phenyl]-piperazin-I-(N-methyl)-thiocarbimidsäuremethylester.

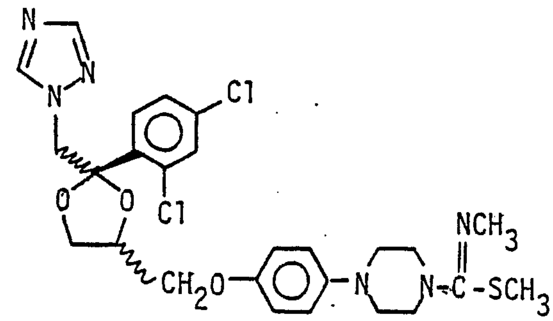

7 g (I2.5 mmol) (±)-cis-4-[4-[[2-(2,4-Dichlorphenyl)-2-(IH-I,2,4-triazol-I-ylmethyl)-I,3-dioxolan-4-yl]-methoxy]-phenyl]-piperazin-I-((N-methyl)-carbothiamid werden in 2OO ml I,2-Dichlorethan gelöst und tropfenweise mit 2,7 g (I8.6 mmol) Methyljodid versetzt. Man erhitzt 4 h auf 4O° C, wäscht das Reaktionsgemisch mit insgesamt 2OO ml IO%-iger wäßriger Kaliumcarbonatlösung und entfernt das Lösungsmittel im Vakuum. Der Rückstand wird aus tert.-Butyl methylether kristallisiert.

Ausbeute: 6.9 g (96 % d.Th.)
Gelblich gefärbtes Pulver vom Schmelzpunkt 7I - 73° C.

Rf: O.5O (Kieselgel: Ethylacetat/Methanol/Triethylamin 9O/5/5)

$C_{26}H_{30}Cl_2N_6O_3S$ (M: 577.5)

| | |
|---|---|
| $^1$H-NMR-Daten: | δ = 2.29 (s) 3 H, |
| ($CDCl_3$, TMS als | 3.09 (m) 4 H, |
| interner Standard) | 3.19 (s) 3 H, |
| | 3.40 (m) 5 H, |
| | 3.80 (m) 3 H, |
| | 4.33 (m) 1 H, |
| | 4.73 (s) 2 H, |
| | 6.83 (m) 4 H, |
| | 7.1 - 7.7 (m) 3 H, |
| | 7.88 1 H, |
| | 8.21 1 H ppm. |

Herstellung von (±)-cis-4-[4-[[2-(2,4-Dichlorphenyl)-2-(lH-l,2,4-triazol-l-ylmethyl)-1,3-dioxolan-4-yl]-methoxy]-phenyl]-piperazin-l-(N-methyl)-carbothiamid.

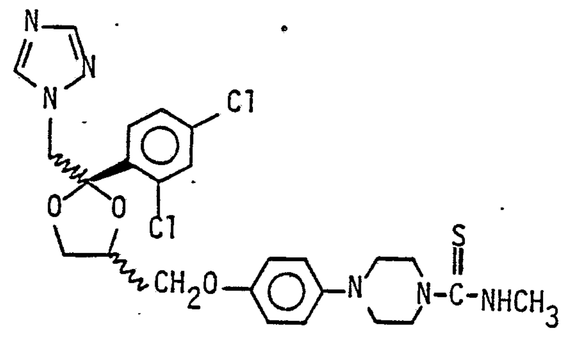

9.8 g (20 mmol) (±)-cis-1-[4-[[2-(2,4-Dichlorphenyl)-2-(lH-l,2,4-triazol-l-ylmethyl)-l,3-dioxolan-4-yl]-methoxy]-phenyl]-piperazin in 50 ml Dichlormethan werden bei RT mit l.5 g (20 mmol) Methylisothiocyanat versetzt.

Nach l h engt man im Vakuum ein und kristallisiert den Rückstand aus Ethylacetat um.

Ausbeute: 9.5 g (85 % d.Th.)

Farbloser Feststoff vom Schmelzpunkt l5l - l52° C.

Rf: 0.55 (Kieselgel; Ethylacetat/Methanol 80/20)

$C_{25}H_{28}Cl_2N_6O_3S$ (M: 563,5l)

| | |
|---|---|
| $^1H$-NMR-Daten: | $\delta$ = 3.17 (m) 7 H, |
| ($CDCl_3$, TMS als | 3.4 - 4.2 (m) 8 H |
| interner Standard) | 4.39 (m) 1 H, |
| | 4.83 (s) 2 H, |
| | 6.0 1 H, austauschbar mit $D_2O$, |
| | 6.88 (m) 4 H, |
| | 7.2 - 7.7 (m) 3 H, |
| | 7.94 (s) 1 H, |
| | 8.27 (s) 1 H ppm. |

**Ansprüche**

1. 1,4-Piperazinverbindungen der allgemeinen Formel I

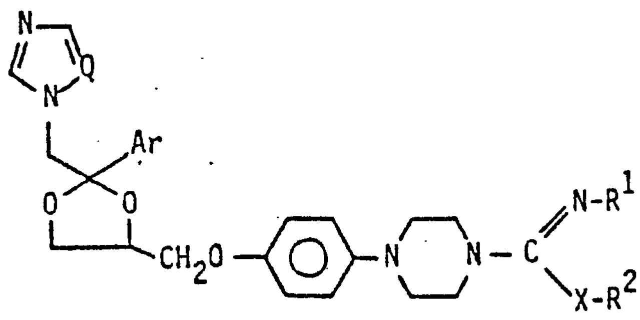

(I)

in der
Q für CH oder N steht,
Ar eine unsubstituierte oder mit bis zu 2 Halogenatomen substituierte Phenylgruppe bedeutet,
X für ein Sauerstoff-oder Schwefelatom steht,
$R^1$ ein Wasserstoffatom oder einen Niedrigalkylrest bedeutet und
$R^2$ einen $C_1$-$C_{10}$-Alkyl-, Cycloalkyl-, $C_1$-$C_{10}$-Alkenyl-, Halogen-$C_1$-$C_{10}$-alkyl-, Hydroxy-$C_1$-$C_{10}$-alkyl-, $C_1$-$C_{10}$-Alkoxy-$C_1$-$C_{10}$-alkyl-, Di-$C_1$-$C_{10}$-alkylamino-$C_1$-$C_{10}$-alkyl-, Ar-Niedrigalkyl-oder Arylrest, der entweder unsubstituiert oder mit l bis 3 Substituenten aus der Gruppe Halogenatome, Niedrigalkyl-und Niedrigalkoxygruppen substituiert ist, bedeutet,
sowie die physiologisch annehmbaren Hydrate und Salze davon.

2. 1,4-Piperazinverbindungen nach Anspruch 1, dadurch **gekennzeichnet,** daß X für ein Sauerstoffatom und $R^1$ für ein Wasserstoffatom stehen, sowie die physiologisch annehmbaren Hydrate und Salze davon.

3. (±)-cis-4-[4-[[2-(2,4-Dichlorphenyl)-2-(1H-1-,2,4-triazol-1-ylmethyl)-1,3-dioxolan-4-yl]-methoxy]-phenyl]-piperazin-1-carbimidsäure-(2,2,2-trifluorethyl)-ester und dessen physiologisch verträgliche Säureadditionssalze.

4. (±)-cis-4-[4-[[2-(2,4-Dichlorphenyl)-1-(1H-1,2,4-triazol-1-ylmethyl)-1,3-dioxolan-4-yl]-methoxy]-phenyl]-piperazin-1-carbimidsäuremethylester und dessen physiologisch verträgliche Säureadditionssalze.

5. (±)-cis-4-[4-[[2-(2,4-Dichlorphenyl)-2-(1H-1-,2,4-triazol-1-ylmethyl)-1,3-dioxolan-4-yl]-methoxy]-phenyl]-piperazin-1-carbimidsäure-(4-methoxyphenyl)-ester und dessen physiologisch verträgliche Säureadditionssalze.

6. Verfahren zur Herstellung von 1,4-Piperazinverbindungen der allgemeinen Formel I

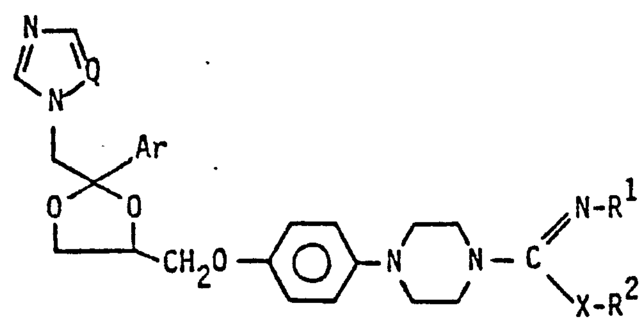

(I)

in der
Q für CH oder N steht,
Ar eine unsubstituierte oder mit bis zu 2 Halogenatomen substituierte Phenylgruppe bedeutet,
X für ein Sauerstoff-oder Schwefelatom steht,
$R^1$ ein Wasserstoffatom oder einen Niedrigalkylrest bedeutet und
$R^2$ einen $C_1$-$C_{10}$-alkyl-, Cycloalkyl-, $C_1$-$C_{10}$-Alkenyl-, Halogen-$C_1$-$C_{10}$-alkyl-, Hydroxy-$C_1$-$C_{10}$-Alkyl-, $C_1$-$C_{10}$-Alkoxy-$C_1$-$C_{10}$-alkyl-, Di-$C_1$-$C_{10}$-alkylamino-$C_1$-$C_{10}$-alkyl-, Ar-Niedrigalkyl-oder Arylrest, der entweder unsubstituiert oder mit 1 bis 3 Substituenten aus der Gruppe Halogenatome, Niedrigalkyl-und Niedrigalkoxygruppen substituiert ist, bedeutet,

sowie der physiologisch annehmbaren Hydrate und Salze davon, dadurch **gekennzeichnet,** daß man

a) zur Herstellung von Verbindungen, bei denen $R^1$ für Wasserstoff steht, $R^2$, Q, Ar und X die oben angegebenen Bedeutungen haben,

$a_1$) eine Verbindung der allgemeinen Formel II

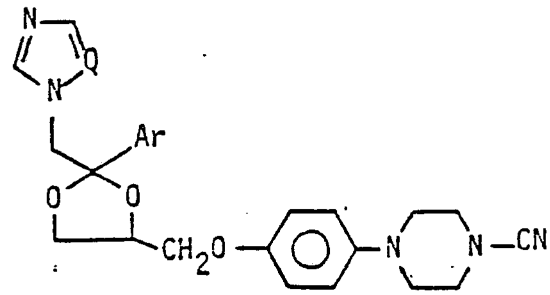

(II)

in der Q und Ar die oben angegebenen Bedeutungen haben, mit einer Verbindung der allgemeinen Formel III

$R^2XH$ (III)

worin $R^2$ und X die oben angegebenen Bedeutungen haben, umsetzt oder

$a_2$) eine Verbindung der allgemeinen Formel IV

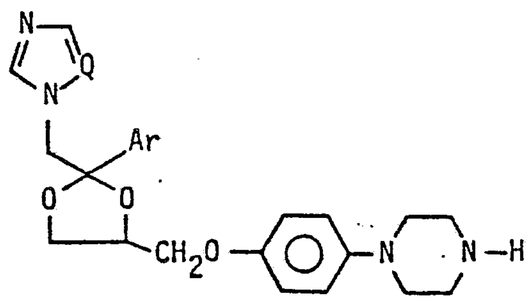

(IV)

worin Q undm Ar die oben angegebenen Bedeutungen haben, mit einer Verbindung der allgemeinen Formel V

$R^2XCN$ (V)

worin $R^2$ und X die oben angegebenen Bedeutungen haben, umsetzt
oder daß man

b) zur Herstellung von Verbindungen der allgemeinen Formel I, bei denen $R^1$ für Wasserstoff steht, $R^2$ wie oben definiert ist, jedoch mit der Maßgabe, daß $R^2$ nicht für einen Arylrest steht, X für ein Schwefelatom steht und Q und Ar die oben angegebenen Bedeutungen haben, eine Verbindung der allgemeinen Formel IV

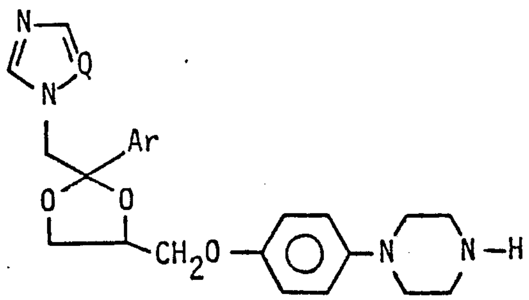

(IV) .

worin Q und Ar die oben angegebenen Bedeutungen haben, mit einer Verbindung der allgemeinen Formel VI

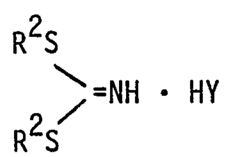

(VI)

worin $R^2$ die oben angegebene Bedeutung hat und HY für eine anorganische Säure steht, umsetzt

oder daß man

c) zur Herstellung von Verbindungen der allgemeinen Formel I, bei denen $R^1$, $R^2$, Q und Ar die oben angegebenen Bedeutungen haben und X für ein Schwefelatom steht, eine Verbindung der allgemeinen Formel VII

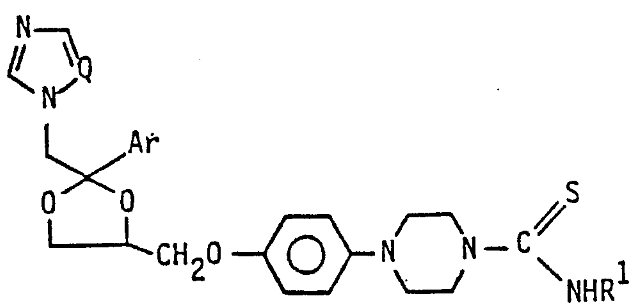

(VII)

worin Q, Ar und $R^1$ die oben angegebenen Bedeutungen haben, mit einer Verbindung der allgemeinen Formel VIII

$R^2Z$ (VIII)

worin $R^2$ die oben angegebene Bedeutung hat und Z für eine Austrittsgruppe steht, umsetzt

oder daß man

d) zur Herstellung von Verbindungen der allgemeinen Formel I, bei denen $R^1$ für ein Wasserstoffatom steht, X für ein Sauerstoffatom steht und $R^2$, Q und Ar die oben angegebenen Bedeutungen haben, eine Verbindung der allgemeinen Formel IX

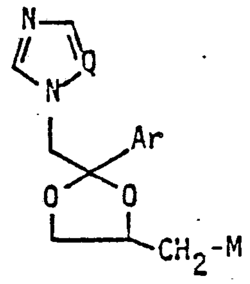

(IX)

worin Q und Ar die oben angegebenen Bedeutungen haben und M den Methansulfonsäure-oder Toluolsulfonsäureester bedeutet,

mit einer Verbindung der allgemeinen Formel X

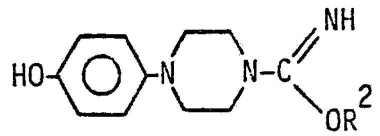

(X)

worin $R^2$ die oben angegebene Bedeutung hat, umsetzt

oder daß man

e) zur Herstellung von Verbindungen der allgemeinen Formel I, bei denen $R^1$ für einen Niedrigalkylrest steht, X für ein Sauerstoffatom steht und $R^2$, Q und Ar wie oben definiert sind, eine Verbindung der allgemeinen Formel Ia

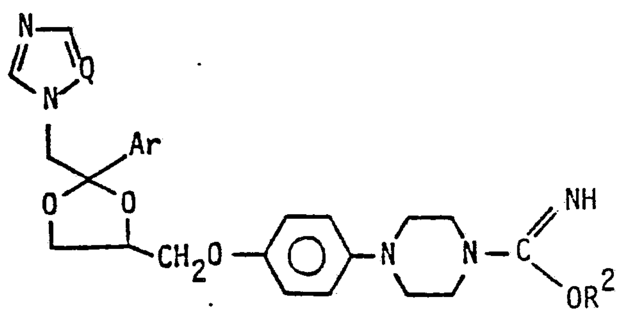

(Ia)

worie Q, Ar und $R^2$ die oben angegebenen Bedeutungen haben, mit einer Verbindung der allgemeinen Formel XI

$R^1Z$ (XI)

worin $R^1$ für einen Niedrigalkylrest steht und Z eine Austrittsgruppe bedeutet, umsetzt

und gegebenenfalls

f) die in den Stufen a) bis e) erhaltenen Verbindungen in ihr physiologisch annehmbares Salz umsetzt.

7. Arzneimittel, dadurch **gekennzeichnet** , daß es eine Verbindung nach den Ansprüchen 1 bis 5 und mindestens einen inerten, pharmazeutisch annehmbaren Träger oder ein inertes, pharmazeutisch annehmbares Verdünnungsmittel enthält.

Patentansprüche für folgende Vertragsstaaten: ES and GR

1. Verfahren zur Herstellung von 1,4-Piperazinverbindungen der allgemeinen Formel I

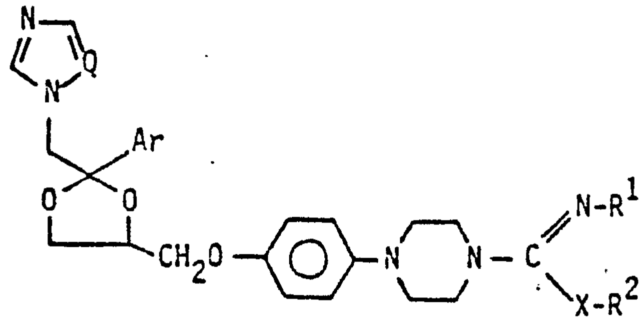

(I)

in der Q für CH oder N steht,
Ar eine unsubstituierte oder mit bis zu 2 Halogenatomen substituierte Phenylgruppe bedeutet,
X für ein Sauerstoff-oder Schwefelatom steht,
$R^1$ ein Wasserstoffatom oder einen Niedrigalkylrest bedeutet und $R^2$ einen $C_1$-$C_{10}$-Alkyl-, Cycloalkyl-, $C_1$-$C_{10}$-Alkenyl-, Halogen-$C_1$-$C_{10}$-alkyl-, Hydroxy-$C_1$-$C_{10}$-alkyl-, $C_1$-$C_{10}$-Alkoxy-$C_1$-$C_{10}$-alkyl-, Di-$C_1$-$C_{10}$-alkylamino-$C_1$-$C_{10}$-alkyl-, Ar-Niedrigalkyl-oder Arylrest, der entweder unsubstituiert oder mit 1 bis 3 Substituenten aus der Gruppe Halogenatome, Niedrigalkyl-und Niedrigalkoxygruppen substituiert ist, bedeutet,
sowie der physiologisch annehmbaren Hydrate und Salze davon, dadurch **gekennzeichnet**, daß man

a) zur Herstellung von Verbindungen, bei denen $R^1$ für Wasserstoff steht, $R^2$, Q, Ar und X die oben angegebenen Bedeutungen haben,

$a_1$) eine Verbindung der allgemeinen Formel II

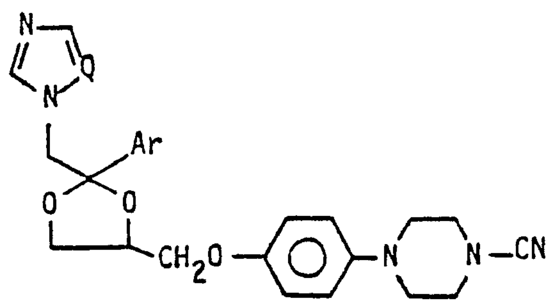

(II)

in der Q und Ar die oben angegebenen Bedeutungen haben, mit einer Verbindung der allgemeinen Formel III

$R^2XH$ (III)

worin $R^2$ und X die oben angegebenen Bedeutungen haben, umsetzt
oder

$a_2$) eine Verbindung der allgemeinen Formel IV

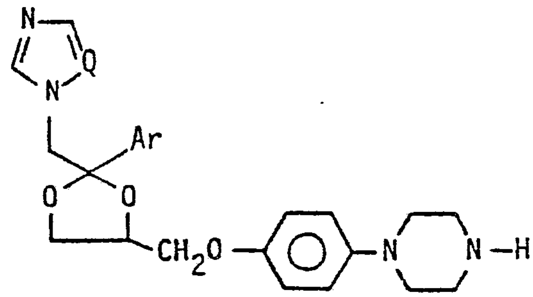

(IV)

worin Q und Ar die oben angegebenen Bedeutungen haben, mit einer Verbindung der allgemeinen Formel V

$R^2XCN$ (V)

worin $R^2$ und X die oben angegebenen Bedeutungen haben, umsetzt
oder daß man

b) zur Herstellung von Verbindungen der allgemeinen Formel I, bei denen $R^1$ für Wasserstoff steht, $R^2$ wie oben definiert ist, jedoch mit der Maßgabe, daß $R^2$ nicht für einen Arylrest steht, X für ein Schwefelatom steht und Q und Ar die oben angegebenen Bedeutungen haben, eine Verbindung der allgemeinen Formel IV

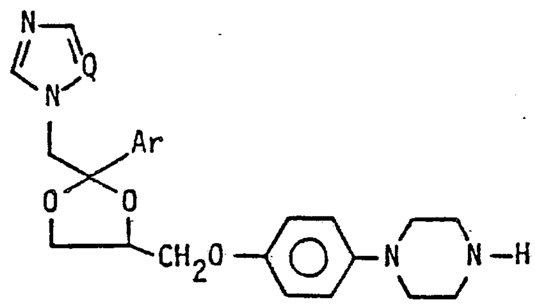

(IV)

worin Q und Ar die oben angegebenen Bedeutungen haben, mit einer Verbindung der allgemeinen Formel VI

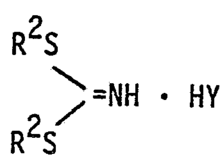

(VI)

worin $R^2$ die oben angegebene Bedeutung hat und HY für eine anorganische Säure steht, umsetzt

oder daß man

c) zur Herstellung von Verbindungen der allgemeinen Formel I, bei denen $R^1$, $R^2$, Q und Ar die oben angegebenen Bedeutungen haben und X für ein Schwefelatom steht, eine Verbindung der allgemeinen Formel VII

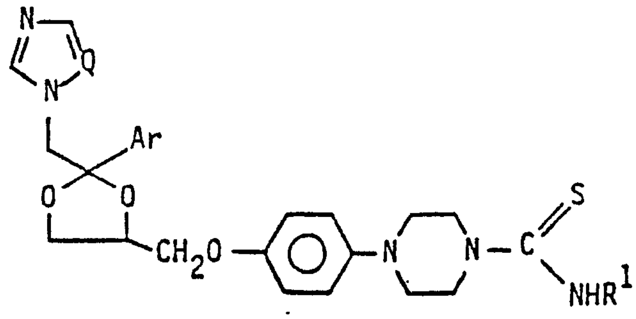

(VII)

worin Q, Ar und R¹ die oben angegebenen Bedeutungen haben, mit einer Verbindung der allgemeinen Formel VIII

$R^2Z$ (VIII)

worin $R^2$ die oben angegebene Bedeutung hat und Z für eine Austrittsgruppe steht, umsetzt oder daß man

d) zur Herstellung von Verbindungen der allgemeinen Formel I, bei denen $R^1$ für ein Wasserstoffatom steht, X für ein Sauerstoffatom steht und $R^2$, Q und Ar die oben angegebenen Bedeutungen haben, eine Verbindung der allgemeinen Formel IX

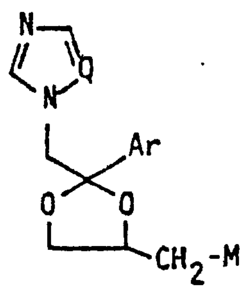

(IX)

worin Q und Ar die oben angegebenen Bedeutungen haben und M den Methansulfonsäure-oder Toluolsulfonsäureester bedeutet, mit einer Verbindung der allgemeinen Formel X

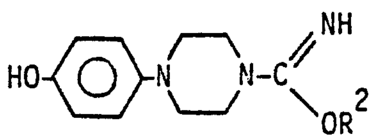

(X)

worin $R^2$ die oben angegebene Bedeutung hat, umsetzt

oder daß man

e) zur Herstellung von Verbindungen der allgemeinen Formel I, bei denen $R^1$ für einen Niedrigalkylrest steht, X für ein Sauerstoffatom steht und $R^2$, Q und Ar wie oben definiert sind, eine Verbindung der allgemeinen Formel Ia

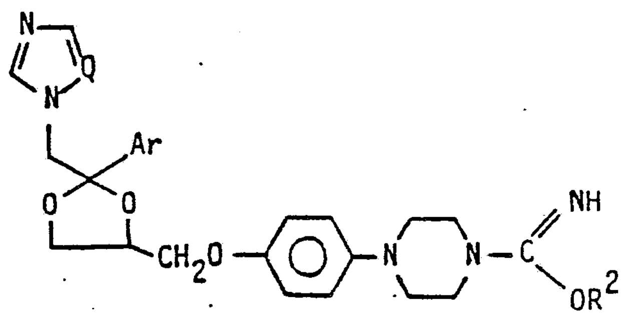

(Ia)

worin Q, Ar und $R^2$ die oben angegebenen Bedeutungen haben, mit einer Verbindung der allgemeinen Formel XI

$R^1Z$ (XI)

worin $R^1$ für einen Niedrigalkylrest steht und Z eine Austrittsgruppe bedeutet, umsetzt

und gegebenenfalls

f) die in den Stufen a) bis e) erhaltenen Verbindungen in ihr physiologisch annehmbares Salz umsetzt.

2. Verfahren nach Anspruch 1 zur Herstellung von l,4-Piperazinverbindungen, bei denen X für ein Sauerstoffatom und $R^1$ für ein Wasserstoffatom stehen, sowie der physiologisch annehmbaren Hydrate und Salze davon.

3. Verfahren nach Anspruch 1 zur Herstellung von (±)-cis-4-[4-[[2-(2,4-Dichlorphenyl)-2-(1H-1,2,4-triazol-1-yl-methyl)-1,3-dioxolan-4-yl]-methoxy]-phenyl]-piperazin-1-carbimidsäure-(2,2,2-trifluorethyl)-ester und dessen physiologisch verträglicher Säureadditionssalze.

4. Verfahren nach Anspruch 1 zur Herstellung von (±)-cis-4-[4-[[2-(2,4-Dichlorphenyl)-1-(1H-l,2,4-triazol-1-yl-methyl)-1,3-dioxolan-4-yl]-methoxy]-phenyl]-piperazin-1-carbimidsäuremethylester und dessen physiologisch verträglicher Säureadditionssalze.

5. Verfahren nach Anspruch 1 zur Herstellung von (±)-cis-4-[4-[[2-(2,4-Dichlorphenyl)-2-(1H-1,2,4-triazol-1-yl-methyl)-1,3-dioxolan-4-yl]-methoxy]-phenyl]-piperazin-1-carbimidsäure-(4-methoxyphenyl)-ester und dessen physiologisch verträglicher Säureadditionssalze.